# EUROPEAN PATENT APPLICATION

(11) **EP 1 710 255 A1**
(43) Date of publication of application: **11.10.2006**
(21) Application number: 04820317.8
(22) Date of filing: 10.12.2004
(51) Int. Cl.: C07K 16/28, C12N 15/13, C12N 15/63, C12N 1/15

(54) **MODIFIED ANTIBODIES RECOGNISING RECEPTOR TRIMERS OR HIGHER MULTIMERS**

(30) Priority: 12.12.2003 JP 2003415735
(71) Applicant: CHUGAI SEIYAKU KABUSHIKI KAISHA, Tokyo, 115-8543 (JP)
(72) Inventor: ONO, Koichiro, c/o Chugai Seiyaku Kabushiki Kaisha, Niihari-gun, Ibaraki 3004101 (JP); TSUCHIYA, Masayuki, c/o Chugai Seiyaki K. K., Gotenba-shi, Shizuka 412038 (JP); ORITA, Tetsuro, c/o Chugai Seiyaku K. K., Niihari-gun, Ibaraki 3004101 (JP)
(74) Representative: Bassil, Nicholas Charles
(86) International application number: PCT/JP2004/018507
(87) International publication number: WO 2005/056605

(57) **Abstract**

The present invention provides tandem diabodies and triabodies against TRAIL receptors, wherein the tandem diabodies and triabodies exhibit greater cytotoxicity as single molecules than whole IgG antibodies. The present invention demonstrates that antibodies that enhance polymerization are useful when aiming to induce cell apoptosis *via* receptors such as TRAIL receptors, which require polymerization of receptor molecules on the surface of cell membranes to transduce cell death signals.

## Description

### Technical Field

The present invention relates to antibodies against tumor necrosis factor-related apoptosis-inducing ligand (TRAIL) receptors.

### Background Art

Some types of cytokines such as tumor necrosis factors (TNFs), have been known to enhance apoptosis. Tumor necrosis factor-related apoptosis-inducing ligands (TRAILs) are members of the TNF family. The ligands lead to cell death in cancer cell lines, but do not appear toxic to most normal human tissues (see Patent Document 1 and Non-patent Document 1). TRAIL is currently known to exhibit affinity for five types of receptors. These five receptors are: four membrane-bound receptors: TRAIL-R1 (also called "DR4"; see Non-patent Document 2), TRAIL-R2 (DR5, TRICK2, or killer; see Non-patent Documents 3 to 5), TRAIL-R3 (TRID, DcR1, or LIT; see Non-patent Documents 3, 4, and 6), and TRAIL-R4 (TRUNDD or DcR2; see Non-patent Documents 6 and 7); and one soluble receptor, osteoprotegerin (OPG; see Non-patent Document 8).

Of these, TRAIL-R1 and TRAIL-R2 are known to have a cytoplasmic death domain (DD). DD is a domain involved in the transduction of apoptotic signals. The binding of the ligand TRAIL to TRAIL-R1 or TRAIL-R2 induces trimerization of TRAIL-R1 or TRAIL-R2. FADD/MORT-1 binds to the DD of the trimerized TRAIL-R1 or TRAIL-R2 and functions as an adaptor molecule that recruits caspase 8 to the receptor. As a result, a proteolytic cascade comprising other caspases is initiated, eventually leading to cell death by apoptosis (see Non-patent Document 9). TRAIL-R3 and TRAIL-R4 are so-called decoy receptors, having extracellular domains but no intracellular domain involved in signal transduction. Thus, they do not transmit apoptotic signals. Unlike TRAIL-R1 and TRAIL-R2, which are expressed in tumor cells, TRAIL-R3 and TRAIL-R4 are essentially expressed in normal tissues and not in tumor cells (see Non-patent Documents 3 to 5).

Monoclonal antibodies against TRAIL receptors are also known. Griffith *et al* reported anti-TRAIL-R1 and TRAIL-R2 antibodies that induce apoptosis in TRAIL-sensitive tumor cells, and anti-TRAIL-R2 antibodies that inhibit TRAIL-induced apoptosis (see Non-patent Document 10). Chuntharaopai *et al* reported mouse anti-TRAIL-R1 monoclonal antibodies (mAbs) that induce apoptosis in tumor cells without other foreign linkers (see Non-patent Document 11). Animal models have been used to confirm that antibodies against TRAIL-R1 have therapeutic effects on human breast cancer, colon cancer, uterine cancer, and such, and thus anticancer agents are being developed using these antibodies. Meanwhile, of the monoclonal antibodies against TRAIL-R2, TRA-8 was reported to exhibit anti-tumor activity (see Non-patent Document 12). Pharmaceuticals for treating progressive tumors are also being developed as a clinical application of anti-TRAIL-R2 antibodies.
Patent Document 1: International Patent Application WO 97/01633
Non-patent Document 1: Wiley et al, Immunology, 1995, Vol. 3, p. 673-82
Non-patent document 2: Pan et al, Science, 1997, Vol. 276, p. 111-3
Non-patent Document 3: Pan et al, Science, 1997, Vol. 277, p. 815-8
Non-patent Document 4: Sheridan et al, Science, 1997, Vol. 277, p. 818-21
Non-patent Document 5: Walczak et al, EMBO J., 1997, Vol. 16, p. 5386-97
Non-patent Document 6: Degli-Esposti et al, J. Exp. Med., 1997, Vol. 186, p. 1165-70
Non-patent Document 7: Marsters et al, Curr. Biol., 1997, Vol. 7, p. 1003-6
Non-patent Document 8: Emery et al, J. Biol. Chem., 1998, Vol. 273, p. 14363-7
Non-patent Document 9: Boder et al, Nat. Cell. Biol., 2000, Vol. 2, p. 241-3
Non-patent Document 10: Griffith et al, J.Immunol., 1999, Vol. 162, p. 2597-605
Non-patent Document 11: Chuntharaopai et al, J.Immunol., 2001, Vol. 166, p. 4891-8
Non-patent Document 12: Buchsbaum et al, Clin. Cancer Res., 2003, Vol. 9, p. 3731-41

### Disclosure of the Invention

An objective of the present invention is to provide anti-TRAIL receptor antibodies exhibiting stronger agonistic activity. Another objective of the present invention is to provide antibodies that exhibit agonistic activity against receptors that form trimers or higher multimers. The antibodies provided by the present invention are not limited to anti-TRAIL receptor antibodies.

The present inventors found that the agonistic activities exhibited by minibodies converted from IgGs were stronger than those of the original IgGs. Based on this finding, the present inventors also attempted to prepare minibodies from anti-TRAIL receptor antibodies with the aim of increasing agonistic activities. TRAIL receptors are known to function as trimers. Thus, triabodies with three antigen binding sites were prepared using 2-, 1-, or 0-mer linkers between the heavy chain variable region (VH) and the light chain variable region (VL) of single-chain Fv(scFv); and tandem diabodies forming four antigen binding sites were prepared using 5-, 12-, and 5-mer linkers for sc(Fv)2. The activities of these triabodies and tandem diabodies were then determined. The results showed that the minibodies by themselves exhibited marked cytotoxic activity towards tumor cells expressing the receptor. It is thought that the transduction of apoptotic signals via TRAIL receptor trimers is enhanced by the promotion of TRAIL receptor polymerization on cell membrane surfaces by triabodies or tandem diabodies. This result also suggests the possibility that minibodies such as triabodies or tandem diabodies act in an agonistic manner towards the cell death-inducing TNF receptor family, which includes TNF receptors and Fas receptors, acting as trimers or higher multimers, thereby transducing cell death signals.

More specifically, the present invention relates to:
[1] an antibody that recognizes a tumor necrosis factor-related apoptosis-inducing ligand receptor (TRAIL receptor);
[2] the antibody of [1], which is a minibody;
[3] the antibody of [1] or [2], which comprises three or more antigen binding sites;
[4] the antibody of [3], which comprises three antigen binding sites;
[5] the antibody of [4], wherein three scFv units form a trimer;
[6] the antibody of [5], wherein two of the variable regions in the scFv units are linked together *via* a linker with zero to two amino acids;
[7] the antibody of [6], wherein the linker comprises zero amino acids;
[8] the antibody of [6], wherein the linker comprises one amino acid;
[9] the antibody of [3], which comprises four antigen binding sites;
[10] the antibody of [9], wherein a polypeptide comprising four variable regions forms a dimer;
[11] the antibody of any one of [1] to [10], wherein the TRAIL receptor is TRAIL-R1 or TRAIL-R2;
[12] the antibody of any one of [1] to [11], which induces apoptosis in a cell;
[13] the antibody of [12], wherein the cell is a tumor cell;
[14] an antibody comprising the amino acid sequence of SEQ ID NO: 2;
[15] an antibody comprising the amino acid sequence of SEQ ID NO: 4;
[16] an antibody comprising the amino acid sequence of SEQ ID NO: 6;
[17] an antibody comprising the amino acid sequence of SEQ ID NO: 8;
[18] an antibody that comprises three or more antigen binding sites and induces apoptosis in a cell;
[19] the antibody of [18], which comprises three antigen binding sites;
[20] the antibody of [18], which comprises four antigen binding sites;
[21] the antibody of any one of [18] to [20], wherein the cell is a tumor cell;
[22] a polynucleotide encoding the antibody of any one of [1] to [21];
[23] a polynucleotide that hybridizes to the polynucleotide of [22] under stringent conditions and encodes an antibody with an activity equivalent to that of the antibody of any one of [1] to [21];
[24] a vector carrying the polynucleotide of [22] or [23];
[25] a host cell carrying the polynucleotide of [22] or [23], or the vector of [24]; and
[26] a pharmaceutical composition comprising the antibody of any one of [1] to [21].

### Brief Description of the Drawings

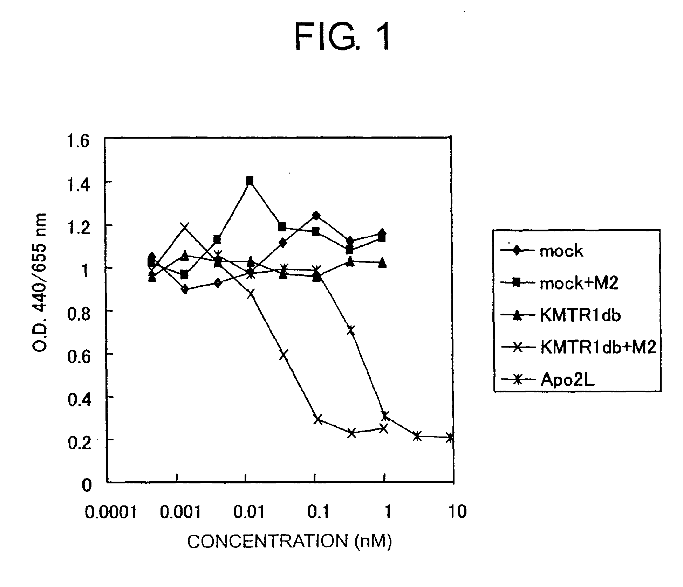

Fig. 1 is a graph showing the results of evaluating diabody cytotoxicity. In the Figure, "mock" indicates the result of introducing the empty pCXND3 vector into COS-7; "mock+M2" indicates the result when M2 antibody is included in the mock assay; "KMTR1 db" indicates the result when a diabody is included in the mock assay; and "KMTR1 db+M2" indicates the result when M2 antibody is included with the KMTR1 db.

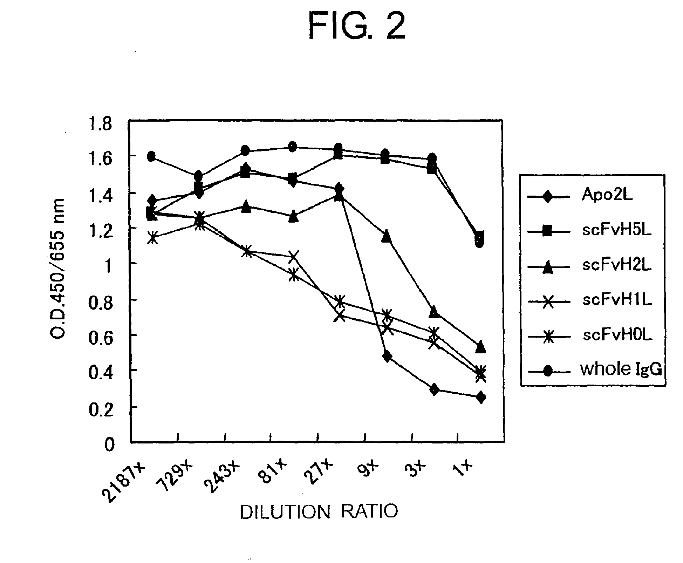

Fig. 2 is a graph showing the results of evaluating triabody and whole IgG cytotoxicity. In this Figure, "ScFvH5L" indicates the assay results obtained by adding a diabody to cells, and "scFvH2L", "scFvH1L", and "scFvH0L" indicate the results obtained by adding triabodies in which the lengths of the linkers between VH and VL are 2-mer, 1-mer, and 0-mer, respectively. "Whole IgG" indicates the results obtained by adding whole IgG.

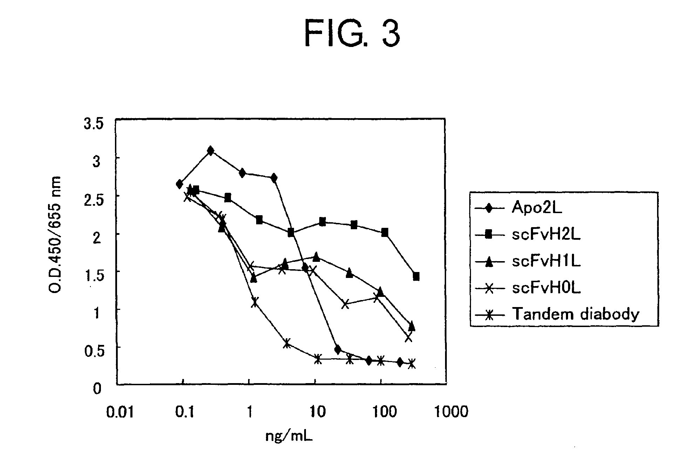

Fig. 3 is a graph showing the results of comparing the cytotoxic activities of triabodies and tandem diabodies. In this Figure, "scFvH2L", "scFvH1L", and "scFvH0" indicate the results obtained by adding triabodies in which the lengths of the linkers between VH and VL are 2-mer, 1-mer, and 0-mer, respectively. "Tandem Diabody" indicates the results obtained by adding a tandem diabody.

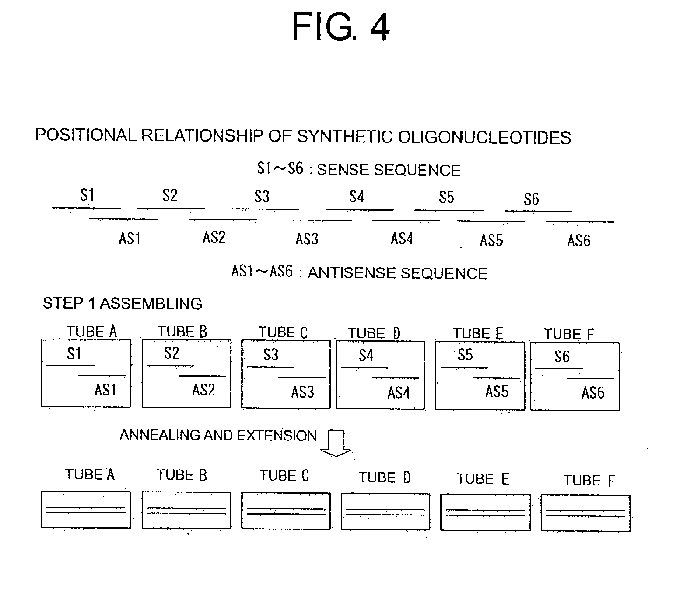

Fig. 4 is a schematic illustration showing the steps when preparing a nucleotide sequence which encodes the full length diabody.

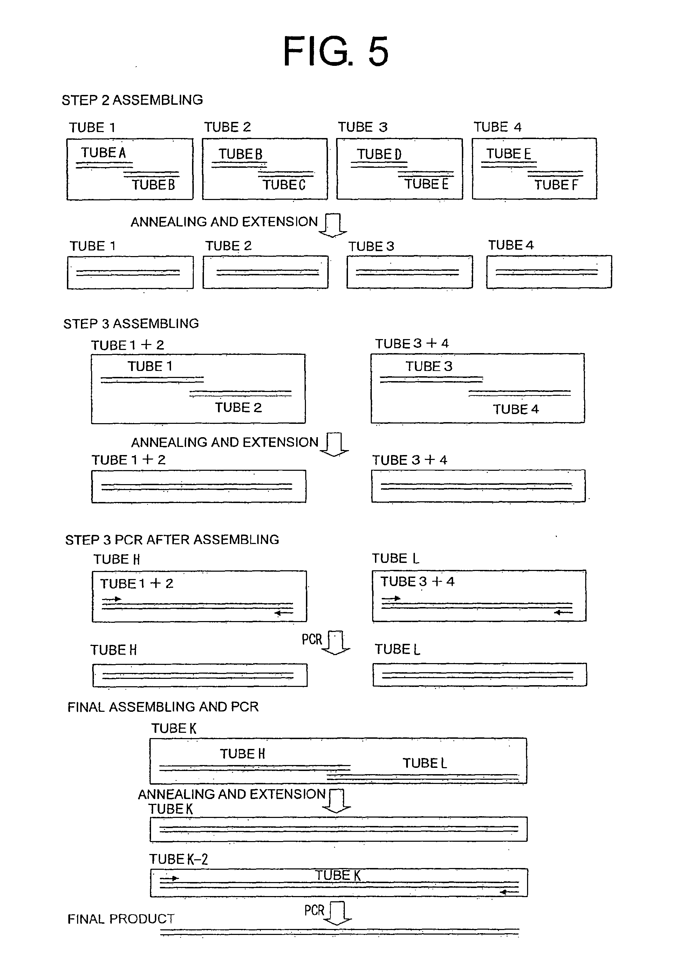

Fig. 5 is a continuation of Fig. 4.

### Best Mode for Carrying Out of the Invention

The following exemplifies the TRAIL receptors and explains the antibodies of the present invention, which are not limited to antibodies against TRAIL receptors, and include all antibodies against receptors that form trimers or higher multimers.

### 1.TRAIL receptor antibodies

The present invention provides antibodies that recognize TNF-related apoptosis-inducing ligand receptors (TRAIL receptors). The antibodies of the present invention that recognize TRAIL receptors can preferably induce cell death (apoptosis or such) in cells expressing TRAIL receptors. Of the TRAIL receptors, TRAIL-R1 and TRAIL-R2 are known to be expressed in tumor cells. Antibodies recognizing TRAIL-R1 or TRAIL-R2 are preferred as the antibodies of the present invention, and of these antibodies, those that induce apoptosis in tumor cells expressing either of these receptors are particularly preferred. The cells in which the antibodies of the present invention induce apoptosis are preferably tumor cells. The tumor cells are not particularly limited, and include, for example, cells of colon cancers, lung cancers, breast cancers, melanomas, colorectal cancers, brain tumors, renal cell carcinomas, bladder cancers, leukemias, lymphomas, T cell lymphomas, multiple myelomas, pancreatic cancers, gastric cancers, cervical cancers, endometrial carcinomas, ovarian cancers, esophageal cancers, liver cancers, head and neck squamous carcinomas, skin cancers, urinary tract cancers, prostate cancers, chorionic carcinomas, pharyngeal cancers, laryngeal cancers, thecomas, arrhenoblastomas, endometrial hyperplasia, endometriosis, embryomas, fibrosarcomas, Kaposi's sarcomas, angiomas, cavernous hemangiomas, hemangioblastomas, retinoblastomas, astrocytomas, neurofibromas, oligodendrogliomas, medulloblastomas, neuroblastomas, gliomas, rhabdomyosarcomas, glioblastomas, osteogenic sarcomas, leiomyosarcomas, goiters, and Wilms tumors.

### (1) TRAIL receptors

In the present invention, a "TRAIL receptor" refers to a receptor to which a TNF-related apoptosis-inducing ligand (TRAIL) binds, and the receptor may be any type of receptor as long as TRAIL binds to it. To date, there are five known types of receptors to which TRAIL binds: TRAIL-1 receptor, TRAIL-2 receptor, TRAIL-3 receptor, TRAIL-4 receptor, and osteoprotegerin (OPG). The antibodies of the present invention may recognize any type of TRAIL receptor. However, antibodies recognizing TRAIL-1 receptor or TRAIL-2 receptor are preferred. The sequence of each TRAIL receptor is known, for example, by referring to sequences registered in GenBank. Anti-TRAIL receptor antibodies of the present invention are preferably those that recognize polypeptides comprising the amino acid sequences of human TRAIL receptors registered under the following GenBank Accession numbers: TRAIL-1 receptor (NP_003835), TRAIL-2 receptor (NP_003833), TRAIL-3 receptor (NP_003832), and TRAIL-4 receptor (NP_003831).

### (2) Antibodies

Herein, the term "antibody" is used in the broadest sense, and the antibodies include monoclonal antibodies, polyclonal antibodies, mutant antibodies (chimeric antibodies, humanized antibodies, minibodies (including antibody fragments), and multi-specific antibodies), as long as they exhibit a desired biological activity. Preferable antibodies are monoclonal antibodies, chimeric antibodies, humanized antibodies, and minibodies such as antibody fragments.

The monoclonal and polyclonal antibodies of the present invention that recognize TRAIL receptors can be prepared by known methods using natural TRAIL receptors as antigens. Alternatively, the antibodies can be prepared using antigenic polypeptides prepared using genetic engineering and based on the known TRAIL receptor sequences described above. The monoclonal antibodies are substantially homogeneous antibody populations that specifically act against a single antigenic determinant (epitope) on an antigen. Thus, monoclonal antibodies are more preferable than polyclonal antibodies, which comprise multiple types of antibodies with specificities to different epitopes. The term "monoclonal antibody" means that a certain antibody shows the properties of a member of a substantially homogeneous antibody population, but does not limit the production methods or such.

Monoclonal antibodies can be obtained, for example, by the following method. First, a TRAIL receptor protein or an antigenic peptide thereof is prepared as a sensitizing antigen to produce antibodies. For example, a polynucleotide comprising the sequence of a gene encoding a TRAIL receptor is inserted into a known expression vector and appropriate host cells are transformed with the expression vector, then the TRAIL receptor protein of interest is purified from the host cells or culture supernatant by known methods. Antibodies are then produced by known techniques using the purified TRAIL receptor protein or a partial peptide of a TRAIL receptor as a sensitizing antigen. In such cases, partial peptides may be obtained by chemical synthesis based on the amino acid sequence of TRAIL receptor. Alternatively, viruses or cells expressing TRAIL receptor on the cell surface can be used as sensitizing antigens. The epitopes in a TRAIL receptor molecule that are recognized by an anti-TRAIL receptor antibody of the present invention are not particularly limited, as long as they are epitopes in the TRAIL receptor molecule. Thus, any fragment can be used as a sensitizing antigen to prepare an anti-TRAIL receptor antibody of the present invention, as long as the fragment comprises an epitope from a TRAIL receptor molecule. The antigens for producing the antibodies of the present invention may be complete antigens with immunogenicity, or incomplete antigens (including hapten) with no immunogenicity.

The mammalian species to be immunized with the sensitizing antigens are not particularly limited; however, they are preferably selected in consideration of compatibility with the parent cells to be used in cell fusion. Rodents, for example, mice, rats, and hamsters, as well as rabbits and monkeys are generally used.

Animals are immunized with sensitizing antigens using known methods. Such standard methods comprise, for example, intraperitoneal or subcutaneous injection of a sensitizing antigen into mammals. Specifically, a sensitizing antigen is suspended and diluted with an appropriate amount of Phosphate-Buffered Saline (PBS), physiological saline, or such. If required, an appropriate amount of a standard adjuvant, for example, Freund's complete adjuvant, is combined with the suspension and the mixture is emulsified. Then, the emulsion is administered to mammals several times at four to 21-day intervals. Appropriate carriers may be used in immunizations using the sensitizing antigen. After a mammal has been immunized by an above method, and the level of desired antibody in the sera is confirmed to be elevated, immune cells are collected from the mammal and subjected to cell fusion.

Herein, spleen cells are a particularly preferable example of immune cells for the above-described purpose. In general, mammalian myeloma cells are used as parent cells for fusion with the immune cells. Various myeloma cell lines are known, and the myeloma cell lines preferably used include, for example, P3 (P3x63Ag8.653) (J. Immnol. (1979) 123:1548-50), P3x63Ag8U.1 (Curr. Topics Microbiol. Immunol. (1978) 81:1-7), NS-1 (Kohler and Milstein, Eur. J. Immunol. (1976) 6:511-9), MPC-11 (Margulies et al., Cell (1976) 8:405-15), SP2/0 (Shulman et al., Nature (1978) 276:269-70), FO (de St. Groth et al., J. Immunol. Methods (1980) 35:1-21), S194 (Trowbridge, J. Exp. Med. (1978) 148:313-23), and R210 (Galfre et al., Nature (1979) 277:131-3). Essentially, the above immune cells can be fused with myeloma cells using known methods, for example, the methods of Kohler and Milstein (Kohler and Milstein, Methods Enzymol. (1981) 73:3-46).

More specifically, for example, cell fusions are carried out in a standard culture liquid in the presence of a cell fusion enhancing agent. For example, polyethylene glycol (PEG), hemagglutinating virus of Japan (HVJ), or such is used as the fusion enhancing agent. If required, an adjuvant such as dimethylsulfoxide can be added to improve fusion efficiency. The ratio of immune cells and myeloma cells can be appropriately determined. For example, the ratio of myeloma cells and immune cells is preferably in the range of 1:1 to 1:10. Culture media that can be used in cell fusions include, for example, RPMI1640 and MEM, which are suitable for proliferating myeloma cell lines. Culture media generally used for these types of cell cultures can also be used appropriately. Furthermore, serum supplements such as fetal calf serum (FCS), may be added to culture media. Cell fusions can be carried out by mixing immune cells with a specified quantity of myeloma cells in a culture liquid, pre-warming a PEG solution (for example, an average molecular weight of about 1000 to 6000) to about 37°C, adding the PEG solution at a concentration of 30% to 60% (w/v), then mixing to generate target fused cells (hybridomas). Then, to remove cell fusion agents and the like, which are unfavorable to hybridoma growth, the following steps are repeated: an appropriate culture medium is sequentially added, the mixture is centrifuged, and the resulting supernatant is removed. The resulting hybridomas can be selected by culturing in a standard selection medium, for example, HAT medium (a culture medium containing hypoxanthine, aminopterin, and thymidine). Culture in the above-described HAT medium is prolonged for a period (typically, several days to several weeks) sufficient to kill cells (non-fused cells) other than the hybridomas of interest. Then, screening for and single clone isolation of hybridomas producing desired antibodies are achieved according to standard limiting dilution methods.

Alternatively, instead of obtaining hybridomas by immunizing nonhuman animals with antigens by the procedures described above, hybridomas producing desired human antibodies with binding activity to TRAIL receptor may be obtained by the *in vitro* sensitizing of human lymphocytes with TRAIL receptor, followed by the fusing of the sensitized lymphocytes with human myeloma cells capable of perpetual division (see Japanese Patent Application Kokoku Publication No. (JP-B) H1-59878 (examined, approved Japanese patent application published for opposition). Alternatively, hybridomas that produce human antibodies against TRAIL receptor may be obtained by administering TRAIL receptor as an antigen to transgenic animals which have the entire repertoire of human antibody genes, and then immortalizing those resulting cells which produce anti-TRAIL receptor antibodies (see International Patent Application WO 94/25585, WO 93/12227, WO92/03918, and WO 94/02602).

Hybridomas producing monoclonal antibodies prepared by the procedures described above can be passaged in conventional culture media and stored in liquid nitrogen for long periods.

Monoclonal antibodies can be obtained from the culture supernatants of hybridomas cultured by conventional methods. Alternative methods comprise transplanting hybridomas to mammals compatible to the hybridomas, allowing the cells to grow, and preparing monoclonal antibodies from ascites of the animal. The former methods are suitable for preparing high purity antibodies, and the latter are suitable for large scale production.

The antibodies of the present invention can also be prepared as recombinant antibodies by using genetic recombination techniques to clone antibody genes from hybridomas, insert the genes into appropriate vectors, and introduce the resulting vectors into hosts (see, for example, Vandamme et al., Eur. J. Biochem. (1990) 192:767-75).

Specifically, an mRNA encoding the variable (V) region of an anti-TRAIL receptor antibody is first isolated from hybridomas which produce that anti-TRAIL receptor antibody. An mRNA can be isolated as follows: total RNA is prepared by known methods, for example, guanidine-ultracentrifugation methods (Chirgwin et al., Biochemistry (1979) 18:5294-9) and AGPC methods (Chomczynski et al., Anal. Biochem. (1987) 162:156-9), and then a desired mRNA is prepared using an mRNA Purification Kit (Pharmacia) or such. Alternatively, it is possible to directly prepare only mRNA by using a QuickPrep mRNA Purification Kit (Pharmacia). Then, a cDNA for the antibody V region is synthesized from the obtained mRNA using reverse transcriptase. cDNA synthesis can be carried out using an AMV Reverse Transcriptase First-strand cDNA Synthesis Kit (Seikagaku Co.) or such. Alternatively, cDNA can be synthesized and amplified by PCR-based 5'-RACE (Frohman et al., Proc. Natl. Acad. Sci. USA (1988) 85:8998-9002; Belyavsky et al., Nucleic Acids Res. (1989) 17:2919-32) using a 5'-Ampli FINDER RACE Kit (Clontech) or such. Then, a DNA fragment of interest is purified from the obtained PCR product and ligated with a vector DNA to prepare a recombinant vector. The recombinant vector is then introduced into a host such as *E. coli,* and colonies of transformed cells are selected. The resulting cells are cultured to produce the desired recombinant antibody. If required, the nucleotide sequence of a DNA of interest is determined by known methods, for example, dideoxynucleotide chain termination methods. Then, the obtained DNA encoding the V region of the antibody of interest is inserted into an expression vector that carries a DNA encoding a desired antibody constant region (C region). The expression vector comprises an expression regulatory region, for example, an enhancer and promotor. The antibody DNA is inserted into the expression vector so that the antibody of the present invention is expressed under the regulation of that expression regulatory region. Then, the antibody is expressed using host cells transformed with the expression vector.

To express an antibody gene, DNAs encoding an antibody heavy chain (H chain) and light chain (L chain) may be separately inserted into different expression vectors and host cells may be co-transformed with these vectors, or host cells may be transformed with a single expression vector carrying both the DNA encoding the H chain and the DNA encoding the L chain (see WO 94/11523).

The antibodies of the present invention include antibodies functionally equivalent to, and with amino acid sequences highly homologous to those of the antibodies of the present invention. In general, the phrase "highly homologous" means an amino acid identity of at least 50% or more, preferably 75% or more, more preferably 85% or more, and still more preferably 95% or more. Polypeptide homology can be determined using algorithms described in the references (Wilbur and Lipman, Proc. Natl. Acad. Sci. USA (1983) 80: 726-30). Such antibodies functionally equivalent to and with high homology to the antibodies of the present invention can be obtained, for example, through hybridization, gene amplification, or such using probes or primers prepared based on the sequence information of DNAs encoding the antibodies of the present invention. Target samples for carrying out hybridization or gene amplification are exemplified by cDNA libraries constructed from cells that are expected to express such antibodies.

Herein, "functionally equivalent" means that a target antibody has biological or biochemical activity equivalent to that of an antibody of the present invention. An antibody's biological and biochemical activities include, for example, binding activities and agonistic activities. Specifically, functional equivalence to an antibody of the present invention can be assessed by determining an antibody's activity of binding to a TRAIL receptor, or its activity of inducing apoptosis *via* a TRAIL receptor. The antibody activity of inducing apoptosis *via* a receptor can be determined, for example, according to methods described in "4. Assessing cytotoxic activity" in the Examples, but is not limited thereto.

### (3) Antibody modification

The antibodies of the present invention include antibodies obtained by the procedures described above and whose amino acid sequences have been modified by amino acid substitutions, deletions, additions, and/or insertions, or chimerization, humanization, and such. Such amino acid sequence modifications such as amino acid substitutions, deletions, additions, and/or insertions, and humanization and chimerization can be achieved by methods known to those skilled in the art. When the antibodies of the present invention are prepared as recombinant antibodies, likewise, the amino acid sequences of the antibody variable and constant regions may also be modified by amino acid substitutions, deletions, additions, and/or insertions, or chimerization, humanization and the like.

As described above, the antibodies of the present invention that recognize a TRAIL receptor may be any antibodies, as long as they have binding activity to a TRAIL receptor. The antibodies are not limited by their origin, shape, or such; however, preferable antibodies are those that bind specifically to a TRAIL receptor. More preferred are agonist antibodies that induce apoptosis *via* a TRAIL receptor. The antibodies of the present invention may be antibodies derived from any animal such as a mouse, human, rat, rabbit, goat, or camel. Furthermore, the antibodies may be modified antibodies, for example, chimeric antibodies, and in particular, modified antibodies that comprise amino acid substitutions in their sequence, such as humanized antibodies. The antibodies may be any type of antibody such as antibody modification products linked with various molecules, antibody fragments, and minibodies.

### (3)-1. Chimeric and humanized antibodies

"Chimeric antibodies" are antibodies prepared by combining sequences derived from different animals. An example is an antibody comprising heavy and light chain variable (V) regions from a mouse antibody and heavy and light chain constant (C) regions from a human antibody. Chimeric antibodies can be prepared by known methods. To obtain such chimeric antibodies, for example, a DNA encoding an antibody V region may be ligated with a DNA encoding a human antibody C region; the resulting ligation product can be inserted into an expression vector; and the construct can be introduced into a host to produce the chimeric antibody.

"Humanized antibodies" are also referred to as reshaped human antibodies, and can be obtained by substituting the complementarity determining region (CDR) of a human antibody for the CDR of an antibody derived from a nonhuman mammal, for example, a mouse. Methods for identifying CDRs are known (Kabat et al., Sequence of Proteins of Immunological Interest (1987), National Institute of Health, Bethesda, Md.; Chothia et al., Nature (1989) 342:877). General genetic recombination techniques for this are also known (see European Patent Application EP 125023; and WO 96/02576). For example, the CDR of a mouse antibody is determined by known methods, and a DNA is prepared so that it encodes an antibody in which the CDR is ligated with the framework region (FR) of a human antibody. A humanized antibody can then be produced using a system that uses conventional expression vectors. Such DNAs can be synthesized by PCR using as primers several oligonucleotides designed to comprise portions that overlap the ends of both the CDR and FR regions (see the method described in WO 98/13388). Human antibody FRs linked *via* CDRs are selected such that the CDRs can form a suitable antigen binding site. If required, amino acids in the FRs of an antibody variable region may be substituted so that the CDRs of the reshaped human antibody can form a suitable antigen binding site (Sato, K. et al., Cancer Res. (1993) 53:851-856). Modifiable amino acid residues in the FRs include portions that directly bind to an antigen *via* non-covalent bonds (Amit et al., Science (1986) 233: 747-53), portions that have some impact or effect on the CDR structure (Chothia et al., J. Mol. Biol. (1987) 196: 901-17), and portions involved in the interaction between VH and VL (EP 239400).

When the antibodies of the present invention are chimeric antibodies or humanized antibodies, the C regions of these antibodies are preferably derived from human antibodies. For example, Cγ1, Cγ2, Cγ3, and Cγ4 can be used for the H chain, while Cκ and Cλ can be used for the L chain. Meanwhile, the human antibody C region may be modified as required to improve antibody or production stability. A chimeric antibody of the present invention preferably comprises a variable region of an antibody derived from a nonhuman mammal and a constant region of a human antibody. A humanized antibody of the present invention preferably comprises CDRs of an antibody derived from a nonhuman mammal and FRs and C regions of a human antibody. The variable regions are described completely in (3)-3. The constant regions of human antibodies comprise specific amino acid sequences, which vary depending on the isotype of the antibody, for example, IgG (IgG1, IgG2, IgG3, and IgG4), IgM, IgA, IgD, and IgE. The constant regions used to prepare the humanized antibodies of the present invention may be the constant regions of antibodies of any isotype. A constant region of human IgG is preferably used, but the constant regions are not limited thereto. The FRs derived from a human antibody, which are used to prepare the humanized antibodies of the present invention, are not particularly limited, and thus may be derived from an antibody of any isotype.

The variable and constant regions of chimeric or humanized antibodies of the present invention may be modified by deletion, substitution, insertion, and/or addition, as long as the antibodies exhibit the same binding specificity as that of the original antibodies.

Chimeric and humanized antibodies using human-derived sequences are expected to be useful when administered to humans for therapeutic purposes or such, since their antigenicity in the human body has been attenuated.

### (3)-2. Minibodies

In a preferred embodiment, the antibodies of the present invention are minibodies. Minibodies are particularly preferable as the antibodies of the present invention because of their *in vivo* kinetic characteristics and low-cost production using *E. coli,* plant cells, or such.

Antibody fragments are one type of minibody. The minibodies include antibodies that comprise an antibody fragment as a partial structural unit. The minibodies of the present invention are not particularly limited by their structure nor their method of production, as long as they have antigen binding activity. In the present invention, the activity of a minibody is greater than that of a whole antibody. Herein, the "antibody fragments" are not particularly limited, as long as they are a portion of a whole antibody (for example, whole IgG). However, the antibody fragments preferably comprise a heavy chain variable region (VH) or a light chain variable region (VL). Examples of preferred antibody fragments are: Fab, F(ab')₂, Fab', and Fv. The amino acid sequence of a VH or VL in an antibody fragment may be modified by substitution, deletion, addition, and/or insertion. Furthermore, some portions of a VH and VL may be deleted, as long as the resulting fragments retain their antigen binding ability. For example, of the antibody fragments described above, "Fv" is a minimal antibody fragment comprising the complete antigen recognition and binding sites. "Fv" is a dimer (VH-VL dimer) consisting of one unit of VH and one unit of VL bound very strongly by non-covalent bonding. The three complementarity determining regions (CDRs) of each variable region interact with each other, thereby forming an antigen binding site on the surface of the VH-VL dimer. Six CDRs confer an antigen binding site to the antibody. However, even one variable region (or half of a Fv comprising only three antigen-specific CDRs) has the ability to recognize and bind to an antigen, although its affinity is lower than that of the complete binding site. Thus, such molecules smaller than Fv are also included in the antibody fragments of the present invention. The variable regions of an antibody fragment may also be chimerized or humanized.

The minibodies preferably comprise both VH and VL. Examples of the minibodies include antibody fragments such as Fab, Fab', F(ab')2, and Fv, and scFv (single-chain Fv), which can be prepared using antibody fragments, (Huston et al., Proc. Natl. Acad. Sci. USA (1988) 85: 5879-83; Plickthun "The Pharmacology of Monoclonal Antibodies" Vol. 113, Resenburg and Moore (eds.), Springer Verlag, New York, pp. 269-315, (1994)); diabodies (Holliger et al., Proc. Natl. Acad. Sci. USA (1993) 90:6444-8; EP 404097; WO93/11161; Johnson et al., Method in Enzymology (1991) 203: 88-98; Holliger et al., Protein Engineering (1996) 9:299-305; Perisic et al., Structure (1994) 2:1217-26; John et al., Protein Engineering (1999) 12(7):597-604; Atwell et al., Mol.Immunol. (1996) 33:1301-12); sc(Fv)2 (Hudson et al, J Immunol. Methods (1999) 231:177-89); triabodies (Journal of Immunological Methods (1999) 231: 177-89); and tandem diabodies (Cancer Research (2000) 60:4336-41).

An antibody fragment can be prepared by treating an antibody with an enzyme, for example, a protease such as papain or pepsin (see Morimoto et al., J. Biochem. Biophys. Methods (1992) 24: 107-17; Brennan et al., Science (1985) 229:81). Alternatively, antibody fragments can also be produced by genetic recombination based on its amino acid sequence.

A minibody with a structure that results from modification of an antibody fragment can be prepared using antibody fragments obtained by enzyme treatment or genetic recombination. Alternatively, after constructing a gene which encodes a whole minibody, and introducing the construct into an expression vector, the minibody may be expressed in appropriate host cells (see, for example, Co et al., J. Immunol. (1994) 152: 2968-76; Better and Horwitz, Methods Enzymol. (1989) 178: 476-96; Pluckthun and Skerra, Methods Enzymol. (1989) 178: 497-515; Lamoyi, Methods Enzymol. (1986) 121: 652-63; Rousseaux et al., Methods Enzymol. (1986) 121: 663-9; Bird and Walker, Trends Biotechnol. (1991) 9: 132-7).

scFv are an example of minibodies whose structure results from modification of antibody fragments. They are single-chain polypeptides that comprise two variable regions linked together *via* a linker or such, as required. The two variable regions in an scFv are typically one VH and one VL, but an scFv may comprise two VH or two VL. In general, scFv polypeptides comprise a linker between the VH and VL domains, thereby forming a paired portion of VH and VL required for antigen binding. A peptide linker comprising ten or more amino acids is generally used as the linker between VH and VL when forming an intramolecular paired portion between VH and VL. However, the linkers of the scFv of the present invention are not limited to such peptide linkers, as long as they do not inhibit the formation of an scFv. To review scFv, see Pluckthun "The Pharmacology of Monoclonal Antibody", Vol. 113 (Rosenburg and Moore ed., Springer Verlag, NY, pp.269-315 (1994)).

Antibodies that form dimers comprising two molecules of scFv linked together by non-covalent bonding are called "diabodies". Diabodies comprise two molecules of scFv and thus have four variable regions. As a result, diabodies have two antigen binding sites. Unlike scFv molecules, which do not form dimers, when aiming to form diabodies the length of the linker between the VH and LH in each scFv molecule is about five amino acids when the linker is a peptide linker. However, the linkers of the scFv that form diabodies of the present invention are not limited to such peptide linkers, as long as they do not inhibit scFv expression and diabody formation.

"sc(Fv)2" are single-chain polypeptide antibodies that are prepared by linking two units of scFv or such *via* a linker or the like, and they comprise four variable regions (Hudson et al, J. Immunol. Methods (1999) 231: 177-89). sc(Fv)2 exhibit particularly high agonistic activity as compared to whole antibodies and other minibodies. Typically, sc(Fv)2 are prepared so as to have two VH-VL pairs in a single molecule and thus to form two antigen binding sites. sc(Fv)2 can be prepared, for example, by linking two units of scFv *via* a linker. sc(Fv)2 typically have the following structure:
[variable region (a)]-linker (A)-[variable region (b)]-linker (B)-[variable region (c)]-linker (C)-[variable region (d)]

Any type of linker can be used. For example, the linkers include peptide linkers and synthetic linkers (see Protein Engineering (1996) 9(3): 299-305). Peptide linkers are preferably used. The length of a peptide linker is not particularly limited, and may be appropriately selected by those skilled in the art according to purpose. Such linkers for use in the minibodies of the present invention are described in detail below in (3)-2-3. The variable regions are also not particularly limited, as long as they have two units of VH and two units of VL. In a particularly preferable example, variable region (a) and variable region (c) are a VH, and variable region (b) and variable region (d) are a VL; linkers (A) and (C) are designed to be short and linker (B) is designed to be long enough to allow formation of pairs between variable regions (a) and (d) and between variable regions (b) and (c), thereby forming two antigen binding sites in a single peptide chain.

In a preferred embodiment, the antibodies of the present invention include three antigen binding sites. There is no upper limit on the number of binding sites. The number is typically within 30 (for example, within ten or five). Preferred antibodies of the present invention comprise three or four antigen binding sites. In general, an antigen binding site consists of a pair of a single heavy chain variable region (VH) and a single light chain variable region (VL) pair. Thus, in general, when comprising three antigen binding sites, an antibody comprises three units of VH and three units of VL; when comprising four antigen binding sites, an antibody comprises four units of VH and four units of VL.

The antibodies of the present invention which comprise three antigen binding sites are not particularly limited by shape. The antibodies may be any type of antibody, as long as they comprise three antigen binding sites. A preferred example is a scFv trimer (a triabody). The antibodies of the present invention which comprise four antigen binding sites are also not particularly limited by shape and so on. The antibodies may be any type of antibody, as long as they comprise four antigen binding sites. A preferred example is a dimer consisting of two units of sc(Fv)2 (a tandem diabody)(Cancer Research (2000) 60: 4336-41).

### (3)-2-1. Triabodies

When forming an scFv trimer (a triabody), scFv molecules may be linked together to form a trimer by non-covalent bonding, or by covalent bonding. Alternatively, a trimer can be formed by combining both non-covalent and covalent bonds within a single molecule to form trimers.

The two variable regions may be linked *via* a linker or such, or directly linked without any linker. The linkers may be any type of linker, including, for example, peptide linkers and synthetic linkers. Peptide linkers are preferably used. The length of a peptide linker is not particularly limited, and may be appropriately selected by those skilled in the art according to purpose. However, it has been reported that triabodies can be formed when the peptide linker length is adjusted to zero to two amino acids (Journal of Immunological Methods (1999) 231: 177-89). Thus, when preparing triabodies, the peptide linkers between variable regions are preferably in the range of zero to two amino acids long, and particularly preferably zero or one amino acids long. In the present invention, a peptide linker of zero amino acids means that the two variable regions are directly linked without a peptide linker.

When preparing a triabody of the present invention, three units of scFv may be linked together as a single-chain polypeptide *via* linkers or such. In this case, the single-chain polypeptide comprises six variable regions. The peptide linker between two scFv is preferably sufficiently long. An antibody prepared as described above can be confirmed to be a triabody by separating the purified polypeptide using gel filtration chromatography and then examining whether a peak for the purified polypeptide is detected at the molecular weight position which corresponds to the trimer. Gel filtration chromatography carriers for use in the present invention include Superdex 200 and Superose 6.

Antibodies comprising three antigen binding sites include, for example, dimers consisting of single-chain polypeptides that comprise three variable regions. In this case, typically, one single-chain polypeptide comprises two heavy chain variable regions (VH) and one light chain variable region (VL), while the other single-chain polypeptide comprises two VL and one VH. Alternatively, one single-chain polypeptide may comprise three heavy chain variable regions (VH), while the other single-chain polypeptide comprises three light chain variable regions.

### (3)-2-2. Tandem diabodies

"sc(Fv)2" are single-chain polypeptide antibodies prepared by linking two units of scFv or such *via* a linker or the like, and they comprise four variable regions. Thus, tandem diabodies, which are dimers consisting of two sc(Fv)2, comprise eight variable regions. The sc(Fv)2 that constitute a tandem diabody typically have the following structure:
[variable region]-linker (1)-[variable region]-linker (2)-[variable region]-linker (3)-[variable region]

In general, of the eight variable regions in a tandem diabody, four are VH and four are VL. The variable regions of sc(Fv)2 that constitute a tandem diabody may comprise four VH and four VL when two sc(Fv)2 molecules are linked together. The variable regions in each molecule can comprise zero to four VH (the remaining variable regions are VL). In this case, the order of the VH and VL units is not particularly limited, and they may be arranged in any order. Thus, a tandem diabody can be constituted by: (1) two sc(Fv)2 that comprise two VH and two VL; (2) an sc(Fv)2 comprising four VH and an sc(Fv)2 comprising four VL; or (3) an sc(Fv)2 comprising three VH and one VL and an sc(Fv)2 comprising three VL and one VH. The tandem diabodies of the present invention include all of the tandem diabodies described above. The linkers for linking the variable regions can be any type of linker. For example, such linkers include peptide linkers and synthetic linkers. Peptide linkers are preferably used. The length of a peptide linker is not particularly limited, and may be appropriately selected by those skilled in the art according to purpose. When forming a tandem diabody, linkers (1) and (3) are preferably designed to be short peptide linkers. For example, these linkers are zero to ten amino acids long, preferably two to eight amino acids, and more preferably four to six amino acids (for example, five amino acids). On the other hand, linker (2) is preferably a long peptide linker. For example, these linkers are 10 to 30 amino acids long, preferably 12 to 20 amino acids, and more preferably 14 to 16 amino acids (for example, 15 amino acids).

When preparing a tandem diabody of the present invention, two units of sc(Fv)2 may be linked together as a single-chain tandem diabody *via* a linker. In this case, the single-chain polypeptide comprises eight variable regions.

An antibody prepared as described above can be confirmed to be a tandem diabody by separating the purified polypeptide using gel filtration chromatography and examining whether a peak for the purified polypeptide is detected at the molecular weight position which corresponds to the dimer. Gel filtration chromatography carriers for use in the present invention include Superdex 200 and Superose 6.

Antibodies comprising four antigen binding sites include, for example, scFv tetramers. All such antibodies are included in the antibodies comprising four antigen binding sites of the present invention.

In the above, examples of antibodies of the present invention that comprise three or four antigen binding sites are shown as preferred embodiments; however, antibodies comprising five or more antigen binding sites can be prepared using the same principles.

### (3)-2-3. Linkers

In the present invention, any type of linker can be used as a minibody linker. It is possible to use, for example, arbitrary peptide linkers that can be introduced using genetic engineering, or synthetic linkers (see, for example, Protein Engineering (1996) 9(3): 299-305).

There is no limit as to the length of the peptide linkers that can be used in the present invention. The length can be appropriately selected by those skilled in the art according to purpose. The length of a peptide linker for an scFV is typically one to 100 amino acids, preferably five to 30 amino acids, and particularly preferably 12 to 18 amino acids (for example, 15 amino acids). Examples of the amino acid sequences of such peptide linkers of the present invention include:
Ser
Gly·Ser
Gly·Gly·Ser
Ser·Gly·Gly
Gly·Gly·Gly·Ser
Ser·Gly·Gly·Gly
Gly·Gly·Gly·Gly·Ser
Ser·Gly·Gly·Gly·Gly
Gly·Gly·Gly·Gly·Gly·Ser
Ser·Gly·Gly·Gly·Gly·Gly
Gly·Gly·Gly·Gly·Gly·Gly·Ser
Ser·Gly·Gly·Gly·Gly·Gly·Gly
(Gly·Gly·Gly·Gly·Ser)n
(Ser·Gly·Gly·Gly·Gly)n
Ala·Ala·Asp·Ala·Ala·Ala·Ala·Gly·Gly·Pro·Gly·Ser
where n is an integer of one or more.

Synthetic linkers (chemical crosslinking agents) which can be used for the antibodies of the present invention include crosslinking agents that are routinely used to crosslink peptides, for example, N-hydroxy succinimide (NHS), disuccinimidyl suberate (DSS), bis(succinimidyl) suberate (BS³), dithiobis(succinimidyl propionate) (DSP), dithiobis(succinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl] sulfone (BSOCOES), and bis[2-(succinimidoxycarbonyloxy)ethyl] sulfone (sulfo-BSOCOES). These crosslinking agents are commercially available.

Three linkers are generally required when linking four antibody variable regions. The linkers may all be the same, or different linkers may be used. Alternatively, the variable regions may be linked without any linker.

### (3)-3. The variable regions of anti-TRAIL receptor antibodies

The variable regions of anti-TRAIL receptor antibodies that can be used to prepare the chimeric antibodies, humanized antibodies, and minibodies of the present invention can be obtained by methods known to those skilled in the art. For example, it is possible to use the variable regions of known antibodies (for example, the antibodies described in WO02/94880). Alternatively, antibodies can be prepared by methods known to those skilled in the art using TRAIL receptors or fragments thereof as immunogens, and variable regions of these prepared antibodies can be used. The sequences of the variable regions of known antibodies or antibodies obtained by known methods can be determined, variable regions can be prepared using genetic engineering techniques, and these resulting variable regions can also be used. There are no limits as to the origin of a variable region or a CDR in a variable region, and they may be derived from any animal. It is possible to use the sequences of antibodies derived from mice, rats, rabbits, or camels, for example.

Furthermore, the amino acids in the variable regions (for example, the FR portions) may be modified. Such amino acid modifications include amino acid substitutions, deletions, additions, and/or insertions, and can be achieved by methods known to those skilled in the art. Specifically, techniques such as site-directed mutagenesis can be used (Hashimoto-Gotoh et al., Gene (1995) 152: 271-5; Zoller and Smith, Methods Enzymol. (1983) 100: 468-500; Kramer et al., Nucleic Acids Res. (1984) 12: 9441-56; Kramer and Fritz, Methods Enzymol. (1987) 154: 350-67; Kunkel, Proc. Natl. Acad. Sci. USA (1985) 82: 488-92; Kunkel, Methods Enzymol. (1988) 85: 2763-6).

When an amino acid residue is mutated in an antibody, the original amino acid is preferably substituted by an amino acid which has side chains with properties similar to those of the original amino acid. For example, based on their side chain properties, amino acids are classified into: hydrophobic amino acids (A, I, L, M, F, P, W, Y, and V), hydrophilic amino acids (R, D, N, C, E, Q, G, H, K, S, and T), amino acids with an aliphatic side chain (G, A, V, L, I, and P), amino acids with a hydroxyl group-containing side chain (S, T, and Y), amino acids with a sulfur atom-containing side chain (C and M), amino acids with a carboxylic acid or amide-containing side chain (D, N, E, and Q), amino acids with a basic side chain (R, K, and H), and amino acids with an aromatic side chain (H, F, Y, and W) (the single letter symbols in parentheses represent amino acids). Side chains with similar properties can be selected based on such classifications. It is already known that a polypeptide with an amino acid sequence in which one or more amino acid residues have been modified by deleting, adding, and/or substituting with other amino acids retains the biological activity of the original polypeptide (Mark et al., Proc. Natl. Acad. Sci. USA (1984) 81: 5662-6; Zoller and Smith, Nucleic Acids Res. (1982) 10: 6487-500; Wang et al., Science (1984) 224: 1431-3; Dalbadie-McFarland et al., Proc. Natl. Acad. Sci. USA (1982) 79: 6409-13). Thus, by introducing appropriate mutations into an antibody of the present invention, an antibody with the same binding specificity as that antibody of the present invention and which is functionally equivalent to the antibody can be prepared, and in some cases, an antibody with improved stability, binding affinity, or such can be prepared.

The present inventors found that in general the agonistic activity of an antibody was different before and after antibody modification. Specifically, even an antibody that does not have agonistic activity before modification may sometimes exhibit agonistic activity when converted to a minibody. Thus, when designing a modified antibody of the present invention, the variable regions of an antibody that binds to a TRAIL receptor but that does not originally have agonistic activity may be used to prepare a modified antibody that exhibits agonistic activity.

In a preferred embodiment, the minibodies of the present invention comprise any one of the following amino acid sequences:
(1) the amino acid sequence shown in SEQ ID NO: 2;
(2) the amino acid sequence shown in SEQ ID NO: 4;
(3) the amino acid sequence shown in SEQ ID NO: 6; and
(4) the amino acid sequence shown in SEQ ID NO: 8.

The antibodies described in (1) to (3) are preferably antibodies with the amino acid sequence shown in any one of SEQ ID NOs: 2, 4, and 6 (ScFvH2L, ScFvH1L, and ScFvH0L, respectively) or multimers thereof, and more preferably trimers (triabodies) of antibodies with the amino acid sequence shown in any one of SEQ ID NOs: 2, 4, and 6.

An antibody described in (4) is preferably an antibody with the amino acid sequence shown in SEQ ID No: 8 (an antibody encoded by pCXND3/KMTR1 Tandab) or a multimer thereof, and more preferably a dimer (a tandem diabody) of an antibody with the amino acid sequence shown in SEQ ID NO: 8.

The nucleotide sequence encoding ScFvH2L is shown in SEQ ID NO: 1; the nucleotide sequence encoding ScFvH1L is shown in SEQ ID NO: 3; the nucleotide sequence encoding ScFvH0L is shown in SEQ ID NO: 5; and the nucleotide sequence encoding pCXND3/KMTR1 Tandab is shown in SEQ ID NO: 7.

The present invention also encompasses antibodies functionally equivalent to antibodies with the sequences shown above. Such antibodies include, for example, mutants of these antibodies.

Specific methods for preparing such functionally equivalent antibodies include, for example, methods for modifying amino acids in the variable regions of the antibodies described above (for example, the FR portions). Such amino acid modifications include amino acid substitutions, deletions, additions, and/or insertions, and can be achieved by methods known to those skilled in the art. Specifically, techniques such as site-directed mutagenesis can be used (Hashimoto-Gotoh et al., Gene (1995) 152:271-5; Zoller and Smith, Methods Enzymol. (1983) 100: 468-500; Kramer et al., Nucleic Acids Res. (1984) 12: 9441-56; Kramer and Fritz, Methods Enzymol. (1987) 154: 350-67; Kunkel, Proc. Natl. Acad. Sci. USA (1985) 82: 488-92; Kunkel, Methods Enzymol. (1988) 85: 2763-6).

When amino acid residues are mutated in antibody variable regions, the original amino acids are preferably substituted by amino acids with side chains with properties similar to those of the original amino acids. For example, amino acids are classified based on their side chain properties into hydrophobic amino acids (A, I, L, M, F, P, W, Y, and V), hydrophilic amino acids (R, D, N, C, E, Q, G, H, K, S, and T), amino acids with an aliphatic side chain (G, A, V, L, I, and P), amino acids with a hydroxyl group-containing side chain (S, T, and Y), amino acids with a sulfur atom-containing side chain (C and M), amino acids with a carboxylic acid or amide-containing side chain (D, N, E, and Q), amino acids with a basic side chain (R, K, and H), and amino acids with an aromatic side chain (H, F, Y, and W) (the single letter symbols in parentheses represent amino acids). Side chains with similar properties can be selected based on such classifications. It is already known that a polypeptide with an amino acid sequence in which one or more amino acid residues has been modified by deleting, adding, and/or substituting with other amino acids retains the biological activity of the original polypeptide (Mark et al., Proc. Natl. Acad. Sci. USA (1984) 81: 5662-6; Zoller and Smith, Nucleic Acids Res. (1982) 10:6487-500; Wang et al., Science (1984) 224: 1431-3; Dalbadie-McFarland et al., Proc. Natl. Acad. Sci. USA (1982) 79: 6409-13). Thus, by introducing appropriate mutations into an antibody of the present invention, an antibody with the same binding specificity as that antibody of the present invention and which is functionally equivalent to the antibody can be prepared, and in some cases, an antibody with improved stability, binding affinity, or such can be prepared.

Antibodies obtained by adding several amino acid residues to the amino acid sequences of the antibodies of the present invention include fusion proteins with other polypeptides. A method for preparing such fusion proteins comprises ligating a DNA encoding an antibody of the present invention with a DNA encoding another peptide or protein in frame, introducing the ligation product to an expression vector, and expressing it in a host. Techniques known to those skilled in the art can be used for this purpose. The peptides or proteins to be fused with the antibodies of the present invention include, for example, known peptides such as FLAG (Hopp et al., Bio/Technology (1988) 6: 1204-10), 6x His consisting of six His (histidine) residues, 10x His, influenza hemagglutinin (HA), human c-myc fragment, VSV-GP fragment, p18HIV fragment, T7-tag, HSV-tag, E-tag, SV40T antigen fragment, lck tag, α-tubulin fragment, B-tag, and Protein C fragment. The proteins to be fused with the antibodies of the present invention also include, for example, glutathione S-transferase (GST), influenza hemagglutinin (HA), immunoglobulin constant region, β-galactosidase, and maltose-binding protein (MBP). Such fusion proteins can be prepared by expressing a fusion DNA prepared by fusing a DNA encoding an antibody of the present invention with a commercially available DNA encoding such a peptide or protein. Since Flag tag has been attached to a triabody or tandem diabody comprising an above-described sequence, this Flag tag moiety may be removed and then an alternative peptide or protein may be fused with it.

### 2. Apoptosis-inducing antibodies comprising three or more antigen binding sites

In the present invention, the Inventors noted that TRAIL receptors functioned as trimers *in vivo.* First, a triabody carrying three antigen binding sites was prepared using a 2-, 1-, or 0-mer linker between the VH and VL of a single-chain Fv(scFv), and a tandem diabody carrying four antigen binding sites was prepared using 5-, 12-, and 5-mer linkers for sc(Fv)2. Their activities were then determined. The results showed that the minibodies by themselves exhibited marked cytotoxic activity against tumor cells expressing the receptor. It is thought that the transduction of apoptotic signals via TRAIL receptor trimers is enhanced by using triabodies or tandem diabodies to promote the polymerization of TRAIL receptors on the surface of cell membranes. Based on this result, it is understood that minibodies with three or more antigen binding sites, such as triabodies and tandem diabodies, may also act against TNF receptor family receptors such as TNF receptor and Fas receptor, which similarly function as trimers and induce cell death, in an agonistic manner and transduce cell death signals more efficiently.

Thus, the present invention provides antibodies with three or more antigen binding sites which induce apoptosis in cells. The antibodies are preferably minibodies with three or more antigen binding sites, which induce apoptosis in cells. Alternatively, such antibodies are preferably antibodies such as triabodies with three antigen binding sites. Alternatively, such antibodies are preferably antibodies such as tandem diabodies with four antigen binding sites.

Cells in which the antibodies of the present invention induce apoptosis are preferably tumor cells. The tumor cells are not particularly limited, and include, for example cells derived from colon cancers, lung cancers, breast cancers, melanomas, colorectal cancers, brain tumors, renal cell carcinomas, bladder cancers, leukemias, lymphomas, T cell lymphomas, multiple myelomas, pancreatic cancers, gastric cancers, cervical cancers, endometrial carcinomas, ovarian cancers, esophageal cancers, liver cancers, head and neck squamous carcinomas, skin cancers, urinary tract cancers, prostate cancers, chorionic carcinomas, pharyngeal cancers, laryngeal cancers, thecomas, arrhenoblastomas, endometrial hyperplasia, endometriosis, embryomas, fibrosarcomas, Kaposi's sarcomas, angiomas, cavernous hemangiomas, hemangioblastomas, retinoblastomas, astrocytomas, neurofibromas, oligodendrogliomas, medulloblastomas, neuroblastomas, gliomas, rhabdomyosarcomas, glioblastomas, osteogenic sarcomas, leiomyosarcomas, goiters and Wilms tumors.

The minibodies against TRAIL receptors are described above in (3)-2 of Section 1. Minibodies against other receptors belonging to the TNF receptor family, for example, TNF receptor and Fas receptor, can be prepared using the same techniques.

### 3. Antibody-encoding polynucleotides

The present invention also provides polynucleotides encoding the antibodies described above in Sections 1 and 2. The polynucleotides of the present invention are not particularly limited, as long as they encode the antibodies of the present invention. The polynucleotides are polymers comprising multiple bases or base pairs, such as deoxyribonucleic acid (DNA) and ribonucleic acid (RNA), and may comprise non-natural bases.

Such polynucleotides of the present invention can be used to express antibodies using genetic engineering techniques. Alternatively, the polynucleotides can be used as probes to screen for antibodies functionally equivalent to the antibodies of the present invention. Specifically, DNAs that hybridize to the polynucleotides under stringent conditions and encode antibodies having activity equivalent to that of the antibodies of the present invention can be obtained using, as probes, polynucleotides encoding antibodies of the present invention or portions thereof, and techniques such as hybridization and gene amplification (for example, PCR). Such DNAs are comprised in the polynucleotide of the present invention. Hybridization techniques (Sambrook et al., Molecular Cloning 2nd ed. (1989) 9.47-9.58, Cold Spring Harbor Lab. press) are well known to those skilled in the art. Hybridization conditions include low stringency conditions, for example. Such low stringency conditions comprise a post-hybridization wash, for example, at 42°C using 0.1x SSC and 0.1% SDS, and preferably at 50°C using 0.1x SSC and 0.1% SDS. More preferable hybridization conditions include highly stringent conditions. Such highly stringent conditions comprise, for example, washing at 65°C using 5x SSC/0.1% SDS. Under these conditions, higher temperatures are expected to more efficiently yield DNAs with greater homology. Factors that may contribute to hybridization stringency include not only temperature and salt concentration but also other multiple factors. Those skilled in the art can achieve stringencies equivalent to those of the conditions described above by selecting appropriate conditions in consideration of these factors.

Antibodies that are encoded by DNAs obtainable by these hybridization and gene amplification techniques and which are functionally equivalent to the antibodies of the present invention generally have high homology to the antibodies of the present invention at the amino acid level.

### 4. Vectors

The present invention also provides vectors carrying the polynucleotides described above in Section 3.

The vectors of the present invention are not particularly limited, and may be any types of vector as long as they carry a polynucleotide of the present invention.

When *E. coli* is used as a host, the vectors of the present invention preferably contain an "ori" responsible for its replication in *E*. *coli* and a marker gene. The "ori" ensures the amplification and mass production of the vector in *E. coli* (for example, JM109, DH5α, HB101, and XL1Blue). The marker gene allows selecting the *E*. *coli* transformants (for example, a drug resistance gene which allows selection by an appropriate drug such as ampicillin, tetracycline, kanamycin, and chloramphenicol). The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs.

In particular, expression vectors are useful as vectors of the present invention. For example, when an expression vector for an antibody of the present invention is expressed in *E*. *coli,* it should have a promoter that allows the efficient expression of the antibody as well as the above characteristics which allow amplification. For example, when *E. coli* such as JM109, DH5α, HB101, or XL1-Blue are used as the host cell, the promoter includes lacZ promoter (Ward et al. (1989) Nature 341:544-546; (1992) FASEB J. 6:2422-2427), araB promoter (Better et al. (1988) Science 240:1041-1043), and T7 promoter. Other examples of the vectors include pGEX-5X-1 (Pharmacia), "QIAexpress system" (QIAGEN), pEGFP, and pET (where BL21, a strain expressing T7 RNA polymerase, is preferably used as the host).

Furthermore, the vectors may comprise a signal sequence for polypeptide secretion. When producing polypeptides into the periplasm of *E. coli,* the pelB signal sequence (Lei *et al. J.* Bacteriol. 169:4379 (1987)) may be used as a signal sequence for polypeptide secretion. For example, calcium chloride methods or electroporation methods may be used to introduce the vector into a host cell.

An expression vector derived from mammals (e.g., pCDNA3 (Invitrogen), pEGF-BOS (Nucleic Acids Res. (1990) 18(17):5322), pEF, pCDM8), insect cells (e.g., "Bac-to-BAC baculovirus expression system" (GIBCO-BRL), pBacPAK8), plants (e.g., pMH1, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as a vector of the present invention.

In order to express proteins in animal cells such as CHO, COS, and NIH3T3 cells, the vector preferably has a promoter necessary for expression in such cells, for example, an SV40 promoter (Mulligan et al. (1979) Nature 277:108), MMLVLTR promoter, EF1α promoter (Mizushima et al. (1990) Nucleic Acids Res. 18:5322), CMV promoter, etc.). It is even more preferable that the vector also carries a marker gene for determining whether the cells were transformed by the vector (for example, a drug-resistance gene selected by a drug such as neomycin and G418). Examples of vectors with such characteristics include pMAM, pDR2, pBK-RSV, pBK-CMV, pOPRSV, and pOP13.

In addition, to stably express a gene and amplify the gene copy number in cells, for example, CHO cells that are defective in the nucleic acid synthesis pathway are introduced with a vector containing a dihydrofolate reductase (DHFR) gene (such as pCHOI) to compensate for the defect, and the vector can be amplified by incubating the cells in the presence of methotrexate (MTX). Alternatively, a COS cell, which carries an SV40 T antigen-expressing gene on its chromosome, can be transformed with a vector containing the SV40 replication origin (for example, pcD) for transient gene expression. The replication origin may be derived from polyoma virus, adenovirus, bovine papilloma virus (BPV), and such. Furthermore, to increase the gene copy number in host cells, the expression vector may contain, as a selection marker, aminoglycoside transferase (APH) gene, thymidine kinase (TK) gene, *E. coli* xanthine guanine phosphoribosyl transferase (Ecogpt) gene, dihydrofolate reductase (dhfr) gene, and such.

### 5. Host cells and hosts and antibody production using thereof

The present invention provides host cells carrying the polynucleotides described above in Section 3 and the vectors described above in Section 4. The host cells are not particularly limited and include, for example, *E. coli* and various animal cells. The host cells may be used, for example, as a production system to produce and express the antibodies of the present invention. *In vitro* and *in vivo* production systems are available for polypeptide production systems. Production systems that use eukaryotic cells or prokaryotic cells are examples of *in vitro* production systems.

Eukaryotic cells that can be used as a host cell include, for example, animal cells, plant cells, and fungal cells. Animal cells include: mammalian cells, for example, CHO (J. Exp. Med. (1995)108, 945), COS, 3T3, myeloma, BHK (baby hamster kidney), HeLa, and Vero; amphibian cells such as *Xenopus laevis* oocytes (Valle, et al. (1981) Nature 291, 338-340); and insect cells (e.g., Sf9, Sf21, and Tn5). In the expression of the antibodies of the present invention, CHO-DG44, CHO-DX11B, COS7 cells, and BHK cells can be suitably used. Among animal cells, CHO cells are particularly preferable for large-scale expression. Vectors can be introduced into a host cell by, for example, calcium phosphate methods, the DEAE-dextran methods, methods using cationic liposome DOTAP (Boehringer-Mannheim), electroporation methods, or lipofection methods.

Plant cells include, for example, Nicotiana tabacum-derived cells known as a protein production system. Calluses can be cultured from these cells to produce the antibodies of the present invention. Known protein production systems are those using fungal cells including yeast cells, for example, cells of genus *Saccharomyces* such as *Saccharomyces cerevisiae* and *Saccharomyces pombe*; and cells of filamentous fungi, for example, genus *Aspergillus* such as *Aspergillus niger.* These cells can be used as a host to produce the antibodies of the present invention.

Bacterial cells can be used in the prokaryotic production systems. Examples of bacterial cells include *Bacillus subtilis* as well as *E. coli* described above, and these cells can be used for producing the antibodies of the present invention.

When producing an antibody using a host cell of the present invention, the polynucleotide encoding an antibody of the present invention may be expressed by culturing the host cells transformed with the expression vector comprising the polynucleotide. The culture can be performed using known methods. For example, when using animal cells as a host, DMEM, MEM, RPMI 1640, or IMDM may be used as the culture medium, and may be used with or without serum supplements such as FBS or fetal calf serum (FCS). Serum-free cultures are also acceptable. The preferred pH is about 6 to 8 during the course of culturing. Incubation is carried out typically at a temperature of about 30 to 40°C for about 15 to 200 hours. Medium is exchanged, aerated, or agitated, as necessary.

On the other hand, production systems using animal or plant hosts may be used as systems for producing polypeptides *in vivo.* For example, a polynucleotide of interest is introduced into an animal or plant and the polypeptide is produced in the body of the animal or plant and then collected. The "hosts" of the present invention includes such animals and plants.

Animals to be used for the production system include mammals or insects. Mammals such as goats, pigs, sheep, mice, and cattle may be used (Vicki Glaser SPECTRUM Biotechnology Applications (1993)). Alternatively, the mammals may be transgenic animals.

For example, a polynucleotide encoding an antibody of the present invention is prepared as a fusion gene with a gene encoding a polypeptide specifically produced in milk, such as the goat β-casein gene. Polynucleotide fragments containing the fusion gene are injected into goat embryos, which are then introduced back to female goats. The desired antibody can be obtained from milk produced by the transgenic goats, which are born from the goats that received the embryos, or from their offspring. Appropriate hormones may be administered to increase the volume of milk containing the antibody produced by the transgenic goats (Ebert et al., Bio/Technology 12: 699-702 (1994)).

Insects such as silkworms, may also be used for producing the antibodies of the present invention. Baculoviruses carrying a polynucleotide encoding an antibody of interest can be used to infect silkworms, and the antibody of interest can be obtained from the body fluids (Susumu et al., Nature 315: 592-594 (1985)).

Plants used for producing the antibodies of the present invention include, for example, tobacco. When tobacco is used, a polynucleotide encoding an antibody of interest is inserted into a plant expression vector, for example, pMON 530, and then the vector is introduced into a bacterium, such as Agrobacterium tumefaciens. The bacteria are then used to infect tobacco such as Nicotiana tabacum, and the desired antibodies can be recovered from the leaves (Ma et al., Eur. J. Immunol. 24: 131-13 8 (1994)).

The resulting antibody may be isolated from the inside or outside (such as the medium and milk) of host cells, and purified as a substantially pure and homogenous antibody. Methods are not limited to any specific method and any standard method for isolating and purifying antibodies may be used. Antibodies may be isolated and purified, by selecting an appropriate combination of, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and others.

Chromatographies include, for example, affinity chromatographies, ion exchange chromatographies, hydrophobic chromatographies, ge1 filtrations, reverse-phase chromatographies, and adsorption chromatographies (Strategies for Protein Purification and Characterization: A Laboratory Course Manual. Ed Daniel R. Marshak et al., Cold Spring Harbor Laboratory Press, 1996). These chromatographies can be carried out using liquid phase chromatographies such as HPLC and FPLC. Examples of the affinity chromatography columns include protein A columns and protein G columns. Examples of the proteins A columns include Hyper D, POROS, and Sepharose F. F. (Pharmacia).

An antibody can be modified freely and peptide portions can be deleted from it by treating the antibody with an appropriate protein modifying enzyme before or after antibody purification, as necessary. Such protein modifying enzymes include, for example, trypsins, chymotrypsins, lysyl endopeptidases, protein kinases, and glucosidases.

The antigens recognized by the antibodies (for example, minibodies and antibodies with three or more antigen binding sites) disclosed in the present invention include not only TRAIL receptors but also other receptors which form trimers or higher multimers. Thus, the present invention includes not only anti-TRAIL receptor antibodies but also antibodies against other receptors which form trimers or higher multimers.

The other receptors which form trimers or higher multimers are not particularly limited. The receptors may be any types of receptor, including, for example, those belonging to the TNF receptor family. Examples of such receptors belonging to the TNF receptor family include: p55-R, CD120a, TNF-R-I p55, TNF-R, TNFR1, TNFAR, TNF-R55, p55TNFR, TNFR60, CD120b, p75, TNF-R, TNF-R-II, TNFR80, TNFR2, TNF-R75, TNFBR, p75TNFR, TNFRSF3, TNFR2-RP, CD18, TNFR-RP, TNFCR, TNF-R-III, OX40, ACT35, TXGP1L, p50, Bp50, CD40, FAS, CD95, APO-1, APT1, DcR3, M68, TR6, HGNC:15888, NHL, DKFZP434C013, KIAA1088, bK3184A7.3, C20orf41, Tp55, S152, CD27, Ki-1, D1S166E, CD30,4-1BB, CD137, ILA,DR4, Apo2, TRAILR-1, DR5, KILLER, TRICK2A, TRAIL-R2, TRICKB, DcR1, TRAILR3, LIT, TRID, DcR2, TRUNDD, TRAILR4, RANK, OPG, OCIF, TR1,DR3, TRAMP, WSL-1, LARD, WSL-LR, DDR3, TR3, APO-3, DR3L, TACI, BAFFR, HVEM, ATAR, TR2, LIGHTR, HVEA, TNFRSF16, p75NTR, BCMA, TNFRSF13, AITR, GITR, TAJ-alpha, TROY, TAJ, TRADE, FLJ14993, RELT, DR6, SOBa, Tnfrh2, 2810028K06Rik, mSOB, and Tnfrh1. (These TNF family receptors are authenticated by the HUGO Gene Nomenclature Committee using the names: tumor necrosis factor receptor superfamily, member 1A; tumor necrosis factor receptor superfamily, member 1B; lymphotoxin beta receptor (TNFR superfamily, member 3); tumor necrosis factor receptor superfamily, member 4; tumor necrosis factor receptor superfamily, member 5; tumor necrosis factor receptor superfamily, member 6; tumor necrosis factor receptor superfamily, member 6b, decoy; tumor necrosis factor receptor superfamily, member 7; tumor necrosis factor receptor superfamily, member 8; tumor necrosis factor receptor superfamily, member 9; tumor necrosis factor receptor superfamily, member 10a; tumor necrosis factor receptor superfamily, member 10b; tumor necrosis factor receptor superfamily, member 10c, decoy without an intracellular domain; tumor necrosis factor receptor superfamily, member 10d, decoy with truncated death domain; tumor necrosis factor receptor superfamily, member 11a, activator of NFKB; tumor necrosis factor receptor superfamily, member 11b (osteoprotegerin); tumor necrosis factor receptor superfamily, member 12-like; tumor necrosis factor receptor superfamily, member 13B; tumor necrosis factor receptor superfamily, member 13C; tumor necrosis factor receptor superfamily, member 14 (herpesvirus entry mediator); nerve growth factor receptor (TNFR superfamily, member 16); tumor necrosis factor receptor superfamily, member 17; tumor necrosis factor receptor superfamily, member 18; tumor necrosis factor receptor superfamily, member 19; tumor necrosis factor receptor superfamily, member 19-like; tumor necrosis factor receptor superfamily, member 21; tumor necrosis factor receptor superfamily, member 22; tumor necrosis factor receptor superfamily, member 23; and the like).

Thus, the present invention includes antibodies against receptors that form trimers or higher multimers, such as receptors belonging to the TNF receptor family. Not only the anti-TRAIL receptor antibodies but also the antibodies against other receptors which form trimers or higher multimers are preferably minibodies or antibodies with three or more antigen binding sites (for example, triabodies and tandem diabodies).

These receptors are not particularly limited, as long as they form trimers or higher multimers. The receptors form, for example, tetramers, pentamers, hexamers, and heptamers. The receptors preferably form trimers or tetramers, and particularly preferably form trimers.

### 6. Pharmaceutical compositions

The present invention provides pharmaceutical compositions comprising the antibodies described above in Sections 1 and 2. When an antibody induces apoptosis in cells (for example, when it is an anti-TRAIL receptor antibody), pharmaceutical compositions comprising that antibody are particularly useful as anticancer agents. Such compositions are expected to show anti-cancer activities by inducing apoptosis in tumor cells, such as in colon cancers, lung cancers, breast cancers, melanomas, colorectal cancers, brain tumors, renal cell carcinomas, bladder cancers, leukemias, lymphomas, T cell lymphomas, multiple myelomas, pancreatic cancers, gastric cancers, cervical cancers, endometrial carcinomas, ovarian cancers, esophageal cancers, liver cancers, head and neck squamous carcinomas, skin cancers, urinary tract cancers, prostate cancers, chorionic carcinomas, pharyngeal cancers, laryngeal cancers, thecomas, arrhenoblastomas, endometrial hyperplasia, endometriosis, embryomas, fibrosarcomas, Kaposi's sarcomas, angiomas, cavernous hemangiomas, hemangioblastomas, retinoblastomas, astrocytomas, neurofibromas, oligodendrogliomas, medulloblastomas, neuroblastomas, gliomas, rhabdomyosarcomas, glioblastomas, osteogenic sarcomas, leiomyosarcomas, goiters and Wilms tumors.

Receptors belonging to the TNF receptor family have been known to be involved in inflammatory diseases (TNFR) such as Crohn's disease and Behcet's disease, and autoimmune diseases such as rheumatoid arthritis (TNFR) and systemic erythematodes (BAFFR), and the like. Therefore, for example, when the antibodies are antibodies against a receptor belonging to the TNF receptor family, pharmaceutical compositions comprising the antibodies are useful for preventing or treating inflammatory diseases, autoimmune diseases, and such.

When an antibody of the present invention is used to prepare pharmaceutical compositions, it is possible to formulate the compositions by methods known to those skilled in the art. For example, such pharmaceutical compositions can be used parenterally, as injections which are sterile solutions or suspensions comprising an antibody along with water or another pharmaceutically acceptable liquid. For example, such compositions may be formulated as unit doses that meet the requirements for the preparation of pharmaceuticals by appropriately combining the antibody with pharmaceutically acceptable carriers or media, specifically with sterile water, physiological saline, a vegetable oil, emulsifier, suspension, detergent, stabilizer, flavoring agent, excipient, vehicle, preservative, binder, or such. In such preparations, the amount of active ingredient is adjusted such that the dose falls within an appropriately pre-determined range.

Sterile compositions for injection can be formulated using vehicles such as distilled water for injection, according to standard protocols for formulation.

Aqueous solutions for injection include, for example, physiological saline and isotonic solutions containing dextrose or other adjuvants (for example, D-sorbitol, D-mannose, D-mannitol, and sodium chloride). Appropriate solubilizers, for example, alcohols (ethanol and such), polyalcohols (propylene glycol, polyethylene glycol, and such), non-ionic detergents (polysorbate 80™, HCO-50, and such), may be used in combination.

Oils include sesame and soybean oils. Benzyl benzoate and/or benzyl alcohol can be used in combination as solubilizers. Buffers (for example, phosphate buffer and sodium acetate buffer), soothing agents (for example, procaine hydrochloride), stabilizers (for example, benzyl alcohol and phenol), and/or antioxidants can also be combined. Prepared injectables are generally filled into appropriate ampules.

The pharmaceutical compositions of the present invention are preferably administered parenterally. For example, the compositions may be injections, transnasal compositions, transpulmonary compositions or transdermal compositions. For example, such compositions can be administered systemically or locally by intravenous injection, intramuscular injection, intraperitoneal injection, subcutaneous injection, or such.

The administration methods can be appropriately selected in consideration of a patient's age and symptoms. The dose of a pharmaceutical composition comprising an antibody or a polynucleotide encoding an antibody may be, for example, from 0.0001 to 1000 mg/kg for each administration. Alternatively, the dose may be, for example, from 0.001 to 100,000 mg per patient. However, the doses are not limited to the ranges described above. The doses and administration methods vary depending on a patient's weight, age, symptoms, and such. Those skilled in the art can select appropriate doses and administration methods in consideration of the factors described above.

Alternatively, if required, the antibodies of the present invention may be formulated in combination with other pharmaceutical ingredients. For example, combinations of antibodies against different types of TRAIL receptors can be used to prepare pharmaceutical compositions. In addition, anti-TRAIL-R2 antibodies whose anti-tumor activity is amplified by their combined use with chemotherapy and/or radiotherapy are known (Buchsbaum et al., Clin. Cancer Res. (2003) 9:3731-41), and thus treatments that use pharmaceutical compositions comprising an antibody of the present invention may be conducted in combination with chemotherapy and radiotherapy. Pharmaceutical ingredients that can be used in such chemotherapy include, for example, preparations of doxorubicin hydrochloride and Paclitaxel. Pharmaceutical ingredients used in combination with the antibodies of the present invention can be formulated together as pharmaceutical preparations, as long as they do not inhibit the activities of the antibodies and pharmaceutical ingredients and can be administered by the same administrative route.

The present invention also relates to methods for inducing cell death by using the antibodies of the present invention. Specifically, the present invention relates to methods for inducing cell death by contacting cells with an antibody of the present invention.

All prior-art documents cited herein are incorporated herein by reference.

### Examples

Hereinafter, the present invention will be explained in more detail with reference to Examples, but is not to be construed as being limited thereto.

### 1. Construction of expression vectors for diabodies, triabodies, tandem diabodies, and whole IgG 1-1. Construction of expression vectors for diabodies

A KMTR1 antibody whose variable region sequence had been determined based on the nucleotide sequence described in patent WO 02/094880 A1 was prepared as a diabody for use in evaluating antibody cytotoxic activity.

The sequence from the adenine (A) at position 81 to the adenine (A) at position 497 of the nucleotide sequence of SEQ ID NO: 32, shown in WO 02/094880 A1, was used as the heavy chain variable region. This sequence comprises the heavy chain signal sequence. The sequence from the guanine (G) at position 123 to the adenine (A) at position 443 of the nucleotide sequence of SEQ ID NO: 34, described in WO 02/094880 A1, was used as the light chain variable region. This sequence is the mature form without the signal sequence.

Gene fragments encoding antibody fragments were designed as follows: For insertion into the expression vector pCXND3, recognition sequences for the restriction enzymes *Eco*RI and *Not*I were attached to the 5' and 3' ends of the gene fragments, respectively. A 5-mer linker sequence comprising Gly-Gly-Gly-Gly-Ser (SEQ ID NO: 10) was attached to the heavy chain variable region (VH) sequence, which comprised an *Eco*RI recognition sequence, Kozak consensus sequence CCACC, and signal sequence in this order. The DNA sequence encoding the linker is 5'-GGT GGA GGC GGA TCG -3' (SEQ ID NO: 9). The linker is followed by the light chain variable region (VL) sequence without the signal sequence, with an epitope tag Flag (Asp-Tyr-Lys-Asp-Asp-Asp-Asp-Lys/SEQ ID NO: 12) sequence then attached. The nucleotide sequence encoding the Flag is 5'-GAC TAC AAG GAT GAC GAC GAT AAG -3' (SEQ ID NO: 11). This is followed by two stop codons, and finally a *Not*I recognition sequence is attached. The nucleotide sequence encoding the designed diabody is shown in SEQ ID NO: 13.

A total of twelve synthetic oligo DNAs were designed to prepare a nucleotide sequence encoding the entire diabody. These oligo DNAs comprise sense and antisense sequences, and are 79 to 103 nucleotides long. The oligo DNAs also comprise sequences complementary to each other, which is essential for links in the assembly. This step is illustrated schematically in Figs. 4 and 5. The nucleotide sequences of the respective synthetic oligo DNAs are shown in SEQ ID NOs: 14 to 25. These SEQ IDs correspond to the names of the oligo DNAs used in the following reactions:
SEQ ID NO: 14: S1;
SEQ ID NO: 15: AS1;
SEQ ID NO: 16: S2;
SEQ ID NO: 17: AS2;
SEQ ID NO: 18: S3;
SEQ ID NO: 19: AS3;
SEQ ID NO: 20: S4;
SEQ ID NO: 21: AS4;
SEQ ID NO: 22: S5;
SEQ ID NO: 23: AS5;
SEQ ID NO: 24: S6; and
SEQ ID NO: 25: AS6.

First, these synthetic oligo DNAs were assembled in three steps. The assembly conditions are described below. The first assembly step was carried out using the following six tubes:
(1) tube A: synthetic DNAs S1 and AS1,
(2) tube B: synthetic DNAs S2 and AS2,
(3) tube C: synthetic DNAs S3 and AS3,
(4) tube D: synthetic DNAs S4 and AS4,
(5) tube E: synthetic DNAs S5 and AS5, and
(6) tube F: synthetic DNAs S6 and AS6.

40 pmol each of the synthetic DNAs was added to each of the tubes. 25 µl of reaction solution that contained a dNTP mix comprising dATP, dGTP, dTTP, and dCTP (250 µM each), 1x TaKaRa pyrobest™ DNA Polymerase buffer, and 1.25 units of TaKaRa pyrobest™ DNA Polymerase was prepared in each tube. The tubes were placed in a thermal cycler Gene Amp PCR System 2400 (Perkin Elmer)(this thermal cycler was used for all reactions described herein below). The thermal cycling was carried out under the following condition: denaturation at 94°C for one minute, followed by five cycles of 94°C for 30 seconds and 72°C for 30 seconds. The second assembly step used the following four tubes:
(1) tube 1: the reaction product of tube A and B,
(2) tube 2: the reaction product of tube B and C,
(3) tube 3: the reaction product of tube D and E, and
(4) tube 4: the reaction product of tube E and F.

10 µl of each reaction product was prepared in each of the tubes. The samples were denatured in the thermal cycler at 94°C for one minute, then five cycles of 94°C for 30 seconds and 72°C for 30 seconds were carried out. The third assembly step used the following two tubes:
(1) tube 1+2: the reaction product of tubes 1 and 2, and
(2) tube 3+4: the reaction product of tubes 3 and 4.

20 µl of each reaction product was prepared in each of the tubes. The samples were denatured at 94°C, then five cycles of 94°C for 30 seconds and 72°C for 30 seconds were carried out.

PCR was carried out after the three assembly steps described above. This PCR used two tubes. The first tube (tube H) contained 50 µl of reaction solution containing 1 µl of the reaction product of tube 1+2, 40 pmol each of the outer primers KMTR1 H1 (SEQ ID NO: 26) and KMTR1 H2 (SEQ ID NO: 27), a dNTP mix comprising dATP, dGTP, dTTP, and dCTP (250 µM each), 1x TaKaRa pyrobest™ DNA Polymerase buffer, and 2.5 units of TaKaRa pyrobest™ DNA Polymerase. The other tube (tube L) contained 50 µl of reaction solution containing 1 µl of the reaction product of tube 3+4, 40 pmol each of the outer primers KMTR1 L1 (SEQ ID NO: 28) and KMTR1 L2 (SEQ ID NO: 29), a dNTP mix comprising dATP, dGTP, dTTP, and dCTP (250 µM each), 1x TaKaRa pyrobest™ DNA Polymerase buffer, and 2.5 units of TaKaRa pyrobest™ DNA Polymerase. The samples in tubes H and L were denatured in the thermal cycler for one minute at 94°C, then 30 cycles of 94°C for 30 seconds and 72°C for 30 seconds were carried out.

Each of the products obtained by the PCR described above were further assembled and amplified by PCR as follows: First, 2.5 µl each of the products obtained in tubes H and L was added to a single tube K, and 50 µl of reaction solution was prepared, containing a dNTP mix comprising dATP, dGTP, dTTP, and dCTP (250 µM each), 1x TaKaRa pyrobest™ DNA Polymerase buffer, and 2.5 units of TaKaRa pyrobest™ DNA Polymerase. After denaturation in the thermal cycler at 94°C for one minute, five cycles of 94°C for 30 seconds and 72°C for 30 seconds were carried out. Then, 1 µl of the reaction product obtained in tube K was added to tube K-2. The prepared tube K-2 contained 50 µl of reaction solution containing 40 pmol each of the outer primers KMTR1 H1 (SEQ ID NO: 26) and KMTR1 L2 (SEQ ID NO: 29), a dNTP mix comprising dATP, dGTP, dTTP, and dCTP (250 µM each), 1x TaKaRa pyrobest™ DNA Polymerase buffer, and five units of TaKaRa pyrobest™ DNA Polymerase. After denaturation in the thermal cycler at 94°C for one minute, 30 cycles of 94°C for 30 seconds and 72°C for 60 seconds were carried out. The reaction products were separated on a 1.2% agarose gel by electrophoresis, and a fragment with a target size of 800 bp was extracted from the gel and purified using a QIAquick Gel Extraction Kit (QIAGEN). Then, the fragment was digested using the restriction enzymes *Eco*RI and *Not*I, and purified using a QIAquick Nucleotide Removal Kit (QIAGEN). The resulting fragment was inserted into the expression vector pCXND3 pretreated with the restriction enzymes *Eco*RI and *Not*I, and the nucleotide sequence was determined. The plasmid comprising the target sequence was named pCXND3/KMTR1#33.

### 1-2. Construction of expression vectors for triabodies

Published documents have reported that when the linker length is appropriately designed in the structure of scFv, scFv can form trimers and thus functions as triabodies with three antigen binding sites (J. Immunol. Methods (1999) 231: 177-89). Based on this finding, three types of triabodies were constructed, each comprising a linker consisting of 2, 1 or 0 Gly amino acid residues, and the resulting triabodies were evaluated. The three types of scFv constituting the respective triabodies were named ScFvH2L, ScFvH1L, and ScFvH0L. Expression vectors for producing each of the triabodies were constructed as described below.

### 1-2-1. Construction of ScFvH2L

The primers ScFv-2S (SEQ ID NO: 30) and ScFv-2A (SEQ ID NO: 31) were designed to hybridize to the diabody expression vector pCXND3/KMTR1#33 so as to flank the region comprising its linker region (Gly-Gly-Gly-Gly-Ser/SEQ ID NO: 10), and so that the linker in the fragment amplified using these primers was the 2-mer "Gly-Gly" linker. The primers were designed so that PCR using pCXND3/KMTR1#33 as a template and the pair of KMTR1 H1 (SEQ ID NO: 26) and ScFv-2A and the pair of ScFv-2S and KMTR1 L2 (SEQ ID NO: 29) produced two fragments, each comprising 18 overlapping nucleotides that allow assembly due to their complementarity.

In tube 2-1, 50 pmol each of the primers KMTR1 H1 and ScFv2A was added to the following reaction solution (hereinafter in Sections 1-2, 1-3-2, and 1-4-2, this is referred to as the "PCR reaction solution"): 50 µl (final volume) of reaction solution containing 100 ng of pCXND3/KMTR1#33 as a template, a dNTP mix comprising dATP, dGTP, dTTP, and dCTP (250 µM each), 1x TaKaRa pyrobest™ DNA Polymerase buffer, and five units of TaKaRa pyrobest™ DNA Polymerase. After tube 2-1, which contained this PCR reaction solution, was denatured in the thermal cycler at 94°C for one minute, 30 cycles of 94°C for 30 seconds and 72°C for 60 seconds were carried out. The reaction products were separated on a 1.2% agarose gel by electrophoresis, and a fragment with a target size of 400 bp was extracted from the gel and purified using a QIAquick Gel Extraction Kit (QIAGEN).

In tube 2-2, 50 pmol each of the primers ScFv2S and KMTR1 L2 was added to the PCR reaction solution, and the total volume was adjusted to 50 µl. In the same way as for tube 2-1, the reaction solution of tube 2-2 was subjected to PCR, and the target 400-bp fragment was purified.

The amplified DNA fragments obtained as the respective reaction products in tube 2-1 and tube 2-2 were assembled and amplified by the following procedure:

1 µl each of the DNA fragments obtained in tube 2-1 and tube 2-2 was added to the reaction solution described below (in section 1-2 shown below, referred to as the "assembly solution"): 50 µl (final volume) of reaction solution containing a dNTP mix comprising dATP, dGTP, dTTP, and dCTP (250 µM each), 1x TaKaRa pyrobest™ DNA Polymerase buffer, and five units of TaKaRa pyrobest™ DNA Polymerase. After the solution in Tube 2 was denatured in the thermal cycler at 94°C for one minute, the DNAs were assembled by five cycles of 94°C for 30 seconds and 72°C for 60 seconds. Then, 0.5 µl each of 100 µM KMTR1 H1 and KMTR1 L2 was added to the reaction solution. After denaturation at 94°C for one minute, the DNA was amplified by 30 cycles of 94°C for 60 seconds and 72°C for 60 seconds. The reaction products were separated on a 1.2% agarose gel by electrophoresis, and a fragment with a target size of 800 bp was extracted from the gel and purified using a QIAquick Gel Extraction Kit (QIAGEN). The purified fragment was digested with the restriction enzymes *Eco*RI and *Not*I, and inserted into the expression vector pCXND3, which had been pre-cleaved with the restriction enzymes *Eco*RI and *Not*I. The nucleotide sequence of the fragment was determined. The plasmid comprising the target sequence was named pCXND3/KMTR1 ScFv2.

### 1-2-2. Construction of ScFvH1L

The primers ScFv-1S (SEQ ID NO: 32) and ScFv-1A(SEQ ID NO: 33) were designed so that the primers hybridize to the expression vector pCXND3/KMTR1#33 for diabody, flanking the region comprising the linker (Gly-Gly-Gly-Gly-Ser/SEQ ID NO: 10) of the vector, and the linker in the fragment amplified using these primers was Gly. The primers were designed so that PCR using pCXND3/KMTR1#33 as a template and the pair of KMTR1 H1 (SEQ ID NO: 26) and ScFv-1A and the pair of ScFv-1S and KMTR1 L2 (SEQ ID NO: 29) produced two fragments, each comprising 18 overlapping nucleotides that allow assembly due to their complementarity.

In tube 1-1, 50 pmol each of the primers KMTR1 H1 and ScFv1A was added to the PCR reaction solution. After denaturation in the thermal cycler at 94°C for one minute, 30 cycles of 94°C for 30 seconds and 72°C for 60 seconds were carried out. The reaction products were separated on a 1.2% agarose gel by electrophoresis, and a fragment with a target size of 400 bp was extracted from the gel and purified using a QIAquick Gel Extraction Kit (QIAGEN).

In tube 1-2, 50 pmol of each of the primers ScFv1S and KMTR1 L2 was added to the PCR reaction solution, and the total volume was adjusted to 50 µl. Like tube 1-1, the reaction solution of tube 1-2 was subjected to PCR, and the target 400-bp fragment was purified.

The amplified DNA fragments obtained as respective reaction products in tube 1-1 and tube 1-2 were assembled and amplified by the following procedure: 1 µl each of the DNA fragments obtained in tube 1-1 and tube 1-2 was added to an assembly solution. The reaction solution in tube 1 was denatured in the thermal cycler at 94°C for one minute, then the DNAs were assembled by five cycles of 94°C for 30 seconds and 72°C for 60 seconds. Then, 0.5 µl each of 100 µM KMTR1 H1 and KMTR1 L2 was added to the tube. After the reaction solution was denatured in the thermal cycler at 94°C for one minute, the DNA was amplified by 30 cycles of 94°C for 60 seconds and 72°C for 60 seconds. The reaction products were separated on a 1.2% agarose gel by electrophoresis, and a fragment with a target size of 800 bp was extracted from the gel and purified using a QIAquick Gel Extraction Kit (QIAGEN). The purified fragment was digested with the restriction enzymes *Eco*RI and *Not*I, and inserted into the expression vector pCXND3, which had been predigested with the restriction enzymes *Eco*RI and *Not*I. The nucleotide sequence of the fragment was determined. The plasmid comprising the target sequence was named pCXND3/KMTR1ScFv1.

### 1-2-3. Construction of ScFvH0L

The primers ScFv-0S (SEQ ID NO: 34) and ScFv-0A (SEQ ID NO: 35) were designed so that they would hybridize to the diabody expression vector pCXND3/KMTR1#33, flanking the region comprising the linker (Gly-Gly-Gly-Gly-Ser/SEQ ID NO: 10) of the vector, and the fragment amplified using these primers contains no linker. The primers were designed so that PCR using pCXND3/KMTR1#33 as a template and the pair of KMTR1 H1 (SEQ ID NO: 26) and ScFv-0 and the pair of ScFv-0S and KMTR1 L2 (SEQ ID NO: 29) produced two fragments, each comprising 18 overlapping nucleotides that allow assembly due to their complementarity.

In tube 0-1, 50 pmol each of the primers KMTR1 H1 and ScFv0A was added to the PCR reaction solution. After denaturation in the thermal cycler at 94°C for one minute, 30 cycles of 94°C for 30 seconds and 72°C for 60 seconds were carried out. The reaction products were separated on a 1.2% agarose gel by electrophoresis, and a fragment with a target size of 400 bp was extracted from the gel and purified using a QIAquick Gel Extraction Kit (QIAGEN).

In tube 0-2, 50 pmol each of the primers ScFv0S and KMTR1 L2 was added to the PCR reaction solution, and the total volume was adjusted to 50 µl. As for tube 0-1, the reaction solution of tube 0-2 was subjected to PCR, and the resulting 400-bp target fragment was purified.

The amplified DNA fragments obtained as reaction products in tube 0-1 and tube 0-2 were assembled and amplified by the following procedure: 1 µl of each of the DNA fragments obtained in tube 0-1 and tube 0-2 were added to the assembly solution in tube 0. The reaction solution was denatured in the thermal cycler at 94°C for one minute, then the DNAs were assembled by five cycles of 94°C for 30 seconds and 72°C for 60 seconds. 0.5 µl each of 100 µM KMTR1 H1 and KMTR1 L2 was added to the mixture. After denaturation at 94°C for one minute, the DNA was amplified by 30 cycles of 94°C for 60 seconds and 72°C for 60 seconds. The reaction products were separated on a 1.2% agarose gel by electrophoresis, and a fragment with a target size of 800 bp was extracted from the gel and purified using a QIAquick Gel Extraction Kit (QIAGEN). The purified fragment was digested with the restriction enzymes *Eco*RI and *Not*I, and inserted into the expression vector pCXND3, which had been predigested with the restriction enzymes *Eco*RI and *Not*I. The nucleotide sequence of the fragment was determined. The plasmid comprising the target sequence was named pCXND3/KMTR1ScFv0.

### 1-3. Construction of expression vectors for tandem diabodies

### 1-3-1. Design of tandem diabodies

When expressed as proteins it has been reported that sc(Fv)2, in which two heavy chain variable regions (VH) and two light chain variable regions (VL) are arranged in tandem in VH-VL-VH-VL order, can form tandem diabodies with a total of four antigen binding sites via the association of paired VH-VL units between the two sc(Fv)2 molecules, if the linkers between the variable regions are appropriately designed (Cancer Research (2000) 60:4336-41).

Herein, the sc(Fv)2 were designed to have three linkers between the variable regions, arranged in the order of: 5-mer, 12-mer, and 5-mer. Specifically, the sequence of the 12-mer linker was the SL sequence (Arg-Ala-Asp-Ala-Ala-Ala-Ala-Gly-Gly-Pro-Gly-Ser/SEQ ID NO: 36) described above, and the 5-mer linkers were Gly-Gly-Gly-Gly-Ser (SEQ ID NO: 10). The amino acid sequence encoded by the constructed vector comprises, in order and from the amino terminus: (VH signal sequence)-(VH)-(5-mer linker)-(VL)-(12-mer linker)-(VH)-(5-mer linker)-(VL)-(Flag tag)-(stop codon).

To obtain a DNA fragment encoding such an sc(Fv)2, PCR was carried out using as a template the diabody expression vector pCXND3/KMTR1#33 prepared in section 1-1. PCR yielded DNA fragment 1, which encodes: (VH signal sequence)-(VH)-(the 5-mer linker)-(VL)-(a portion of the 12-mer linker); and DNA fragment 2, which encodes: (a portion of the 12-mer linker)-(VH)-(the 5-mer linker)-(VL)-(Flag tag)-(stop codon). A DNA fragment encoding an sc(Fv)2 was constructed by ligating these two fragments using the *Sma*I restriction enzyme recognition sequence within the 12-mer linker.

### 1-3-2. Construction of tandem diabodies

The primer KMTR1tanA (SEQ ID NO: 37) has an antisense sequence that comprises a sequence encoding the 12-mer linker sequence, which comprises a *Sma*I recognition sequence after a sequence that hybridizes to the end of the VH of the diabody expression vector pCXND3/KMTR1#33. The primer KMTRItanS (SEQ ID NO: 38) has a sense sequence that comprises a sequence that hybridizes to the end of the VL of the diabody expression vector pCXND3/KMTR1#33 after a sequence encoding the 12-mer linker sequence, which comprises a *Sma*I recognition sequence. Fragments 1 and 2 were amplified using these primers.

In tube #1,50 pmol each of the primers KMTR1 H1 (SEQ ID NO: 26) and KMTR1tanA was added to the PCR reaction solution (described in Section 1-2) comprising pCXND3/KMTR1#33 as a template. After denaturation in the thermal cycler at 94°C for one minute, 30 cycles of 94°C for 30 seconds and 72°C for 60 seconds were carried out. The reaction product was fractionated by electrophoresis in a 1% agarose gel, and a fragment with an expected size of 800 bp was extracted from the gel and purified using a QIAquick Gel Extraction Kit (QIAGEN). This fragment was digested with the restriction enzymes *Eco*RI and *Sma*I, and inserted into the vector pBluescript^{(R)}II (TOYOBO), which had been pre-digested with the restriction enzymes *Eco*RI and *Sma*I. The nucleotide sequence of the fragment was determined. The plasmid comprising the target sequence was named pBS/KMTR1tanFr1.

In tube #2, 50 pmol each of the primers KMTR1 L2 (SEQ ID NO: 29) and KMTRItanS was added to the PCR reaction solution (described in section 1-2) comprising pCXND3/KMTR1#33 as a template. After denaturation in the thermal cycler at 94°C for one minute, 30 cycles of 94°C for 30 seconds and 72°C for 60 seconds were carried out. The reaction product was fractionated by electrophoresis in a 1% agarose gel, and a fragment with an expected size of 800 bp was extracted from the gel and purified using a QIAquick Gel Extraction Kit (QIAGEN). The fragment was digested with the restriction enzymes *Sma*I and *Not*I, and inserted into the vector pBluescript^{(R)}II, which had been pre-digested with the restriction enzymes *Sma*I and *Not*I. The nucleotide sequence of the fragment was determined. The plasmid comprising the target sequence was named pBS/KMTR1tanFr2.

Then, pBS/KMTR1tanFr1 was digested with the restriction enzymes *Eco*RI and *Sma*I, and pBS/KMTR1tanFr2 was digested with the restriction enzymes *Sma*I and *Not*I, respectively. The reaction products were fractionated by electrophoresis in a 1% agarose gel, and fragments with an expected size of 800 bp were extracted from the gel and purified using QIAquick Gel Extraction Kit (QIAGEN). Both fragments were inserted into the expression vector pCXND3, which had been pre-digested with the restriction enzymes *Eco*RI and *Not*I. The plasmid carrying fragments of the target length was named pCXND3/KMTR1 Tandab.

### 1-4. Construction of expression vectors for whole IgG

### 1-4-1. Design of expression vectors for whole IgG

As previously reported in Patent (WO 92/19759), when a fragment comprising a signal sequence and VH is inserted into the expression vector HEF-PMh-gγ1, the H chain of whole IgG in which the human H chain constant region is attached to the VH fragment is expressed under the control of human EF1α promoter. Likewise, when a fragment comprising a signal sequence and VL is inserted into the expression vector HEF-PM1k-gκ, the L chain of whole IgG in which the human L chain constant region is attached to the VL fragment is expressed under the control of human EF1α promoter. Whole IgG can be expressed by co-introducing expression vectors for these H and L chains into COS-7 animal cells or such.

H chain expression vectors can be constructed by the procedure as described below. A DNA sequence stretch encoding the signal sequence and VH is inserted into the diabody expression vector pCXND3/KMTR1#33. Thus, to insert this DNA encoding the signal sequence and VH into the expression vector HEF-PMh-gγ1, it is first necessary to amplify a corresponding partial sequence by PCR using appropriate primers and pCXND3/KMTR1#33 as a template. The amplified sequence may be treated with restriction enzymes if required, and is then inserted into the appropriately treated expression vector HEF-PMh-gγ1.

L chain expression vectors can be constructed by the procedure as described below. The diabody expression vector pCXND3/KMTR1#33 carries VL as an insert, without its signal sequence. However, VL with a signal sequence can be amplified by PCR using pCXND3/KMTR1#33 as a template and an appropriate antisense primer in combination with a sense primer designed and synthesized to allow a nucleotide sequence corresponding to the signal sequence of the KMTR1 antibody L chain, described in Patent (WO 02/094880 A1), to be added to the VL. The fragment amplified as described above may be treated with restriction enzymes if required, and is then inserted into the appropriately treated expression vector HEF-PM1k-gκ. L chain expression vectors can be constructed by the above-described procedures.

### 1-4-2. Construction of expression vectors for whole IgG

The sense primer KMTRVHsp (SEQ ID NO: 39) was designed to hybridize to the amino terminus of the coding sequence of pCXND3/KMTR1#33. A *Hind*III restriction enzyme recognition sequence is added to KMTRVHsp for cloning purposes. The antisense primer KMTRVHap (SEQ ID NO: 40) was designed to hybridize to the carboxyl terminus of the coding sequence of pCXND3/KMTR1#33 and to have a splice donor sequence immediately after the carboxyl terminus. A *Bam*HI restriction enzyme recognition sequence was added to KMTRVHap for cloning purposes. The sense primer KMTRVLsp (SEQ ID NO: 41) was designed to comprise a sequence encoding the KMTR1 VL signal sequence described in Patent (WO 02/094880), and upstream of that a Kozak consensus sequence CCACC and a *Bam*HI restriction enzyme recognition sequence. KMTRVLsp was designed to hybridize to the amino terminus of VL in the coding sequence of pCXND3/KMTR1#33. In addition, *Hind*III restriction enzyme recognition sequence was added to KMTRVLsp for cloning purposes. The antisense primer KMTRVLap (SEQ ID NO: 42) was designed to hybridize to the carboxyl terminus of VL in the coding sequence of pCXND3/KMTR1#33 and to have a splice donor sequence immediately after the carboxyl terminus. KMTRVLap comprises a *Bam*HI restriction enzyme recognition sequence for cloning purposes.

In tube VH, 50 pmol each of the primers KMTRVHsp and KMTRVHap was added to the PCR reaction solution (described in Section 1-2) comprising pCXND3/KMTR1#33 as a template. After denaturation in the thermal cycler at 94°C for one minute, 30 cycles of 94°C for 30 seconds and 72°C for 60 seconds were carried out. The reaction product was fractionated by electrophoresis in a 1% agarose gel, and a fragment with an expected size of 400 bp was extracted from the gel and purified using a QIAquick Gel Extraction Kit (QIAGEN). This fragment was digested with the restriction enzymes *Bam*HI and *Hin*dIII, and inserted into the expression vector HEF-PMh-gγ1, which had been pre-digested with the restriction enzymes *Bam*HI and *Hin*dIII. The nucleotide sequence of the fragment was determined. The plasmid comprising the target sequence was named pHEF-KMTR1VH-gγ1.

In tube VL, 50 pmol each of the primers KMTRVLsp and KMTRVLap was added to the PCR reaction solution (described in Section 1-2) comprising pCXND3/KMTR1#33 as a template. After denaturation in the thermal cycler at 94°C for one minute, 30 cycles of 94°C for 30 seconds and 72°C for 60 seconds were carried out. The reaction product was fractionated by electrophoresis in a 1% agarose gel, and a fragment with an expected size of 400 bp was extracted from the gel and purified using a QIAquick Gel Extraction Kit (QIAGEN). The fragment was digested with the restriction enzymes *Bam*HI and *Hin*dIII, and inserted into the expression vector HEF-PM1k-gκ, which had been pre-digested with the restriction enzymes *Bam*HI and *Hin*dIII. The nucleotide sequence of the fragment was determined. The plasmid comprising the target sequence was named pHEF-KMTR1VL-gκ.

### 2. Expression of diabodies, triabodies, tandem diabodies, and whole IgG

10 µg each of the expression vectors constructed as described in Section 1 was introduced into COS-7 cells by electroporation using Gene Pulser. Specifically, each DNA (10 µg) was added to 0.8 ml aliquots of 1 x 10⁷ cells suspended in PBS, and 1500 V, 25 µF pulses were applied. After ten minutes of recovery at room temperature, the cells treated by electroporation were plated on 30 ml of DMEM (GIBCO BRL) comprising 10% bovine fetal serum (GIBCO BRL). The cells were cultured at 37°C under a 5% CO₂ overnight, and then the medium was removed. The cells were washed four times with PBS, and then 15 ml of CHO-S-SFMII medium (GIBCO BRL) was added to the cells. The cells were then cultured at 37°C under a 5% CO₂ for 72 hours. After the cell debris was removed by centrifugation, the resulting supernatant was filtered with 0.45-µm filter. The culture supernatant obtained was used in cytotoxic activity assays.

### 3. Determining the concentrations of expression products

### 3-1. Determining diabody, triabody, and tandem diabody concentrations

The concentrations of the expressed diabodies, triabodies, and tandem diabodies in the culture supernatants as described in Section 2 were determined on a biosensor BIAcore2000 (BIACORE) using surface plasmon resonance. Flag tag was attatched to the antibodies. Thus, the analysis was carried out using anti-Flag antibody M2 (Sigma). More specifically, the antibodies were immobilized onto a CM5 sensor chip (Biacore) using an amino-coupling method, and surface plasmon resonance signals on the sensor chip were measured using the culture supernatants.

### 3-2. Determining the concentration of whole IgG

The concentrations of expressed IgG in the culture supernatant as described in Section 2 were determined using ELISA. 100 µl of goat anti-human IgG antibody (BioSource) whose concentration had been adjusted to 1 µg/ml using coating buffer (0.1 M NaHCO₃/0.02% NaN₃ (pH9.6)) was added to each well of a 96-well Maxisorp ELISA plate (NUNC), and the plate was incubated for one hour at room temperature to immobilize the antibody. After blocking with 100 µl of dilution buffer (50 mM Tris-HCl, 1 mM MgCl₂, 0.15 M NaCl, 0.05% Tween20, 0.02% NaN₃, and 1% bovine serum albumin (BSA) (pH8.1)), culture supernatants containing expressed whole IgG were serially diluted and a 100-µl aliquot was added to each well. The plate was incubated for one hour at room temperature. After each well was washed, 100 µl of alkaline phosphatase-labeled goat anti-human IgG (BioSource) was added to each well. After one hour of incubation at room temperature, each well was washed and 100 µl of 1 mg/ml substrate solution (Sigma104; p-nitrophenyl phosphate; Sigma) dissolved in substrate buffer (50 mM NaHCO₃/10 mM MgCl₂ (pH9.8)) was added thereto. Absorbance at 405 nm was measured using a Microplate Reader Model 3550 (Bio-Rad). A standard used to determine the concentrations was human IgG1κ (The Binding Site).

### 4. Assessing cytotoxic activity

The biological activities of expressed diabodies, triabodies, and tandem diabodies in the culture supernatants as described in Section 2 were assessed based on their cytotoxic activities. Specifically, cells of the colon cancer cell line COLO 205 (ATCC CCL-222), which have been confirmed to express TRAIL receptor, were plated to 96-well cell culture microplates (FALCON) at a cell density of 7.5 x 10⁴ cells/well, and each culture supernatant was serially diluted with CHO-S-SFMII (GIBCO BRL) and added to each well. As a cross-linker, an anti-Flag antibody M2 (Sigma) was added at the concentration of 10 µg/ml, as required. When assessing cytotoxic activity, the recombinant Apo2L (Sigma), a natural TRAIL ligand, was diluted with CHO-S-SFMII and used as a positive control. The microplates prepared as described above were incubated at 37°C under 5% CO₂ overnight. On the following day, Cell Counting Kit-8 (WAKO), an assay reagent for cell growth/cytotoxicity, was added to each well to allow color development, and then absorbance at 450 nm was measured using a Microplate Spectrophotometer Benchmark Plus™ (Bio-Rad).

Fig. 1 shows the results obtained by the assay for cytotoxic activity of diabodies. The results showed that cell count did not decrease after addition of the diabody alone, suggesting that the diabody has no cytotoxic activity by itself. However, marked cytotoxic activity was detected when M2 antibody was added to crosslink the diabody. This suggested that apoptotic signals are efficiently transmitted when the polymerization of TRAIL receptor on the surface of cell membranes is enhanced. Then, the form in which the single molecule exhibited greatest activity was investigated.

Fig 2 shows the results of the assays for the cytotoxic activities of triabodies and whole IgG. The results showed that neither the diabodies nor whole IgG had marked cytotoxic activity. In contrast, the cell count was dramatically decreased after addition of the triabody, suggesting that the triabody had obvious cytotoxic activity. In particular, the activity was significantly higher when the triabody had the 1-mer or 0-mer linker. In this experiment, whole IgG (IgG1/κ) did not exhibit cytotoxic activity. This result do not agree with that described in Patent (WO 02/094880 A1), showing that the LD50 (lethal dose 50%) of the same antibody in the form of IgG1 was 100 ng/ml for COLO 205 cells. Thus, the COLO 205 cell binding activity of the whole IgG used in this experiment was evaluated using a cell sorter. The result showed that there was a sufficient shift in histograms as compared with the mock, and thus the whole IgG was thought to retain binding activity.

Then, the cytotoxic activity was compared between the triabody and tandem diabody. The results are shown in Fig. 3. This result showed that the activity of the tandem diabody was stronger than that of the triabody, and was equivalent to or greater than that of the natural ligand Apo2L. These results suggest that of the molecules tested, the tandem diabody by itself is the most effective molecule.

As described above, it was demonstrated that tandem diabodies and triabodies are molecular forms that by themselves as single molecules exhibit stronger cytotoxic activities than whole IgG. This is a novel finding suggesting that, when TRAIL receptor is polymerized on the surface of a cell membrane, the activation level of signals inducing cell death varies depending on the degree of polymerization, and as the degree of polymerization increases, the signals inducing cell death are more activated. Such phenomena can also be predicted for the TNF receptor family in general, including Fas receptor and TNF receptor, which transmit cell death signals by the same intracellular signaling mechanism. Thus, it is expected that antibodies comprising three or more antigen binding sites, such as tandem diabodies and triabodies, can be used to induce apoptosis *via* these receptors other than TRAIL receptors.

When converted to minibodies, antibodies can exhibit higher specific activity and their half-lives in blood can be shorter. Therefore, the effective concentration in blood can be controlled easily when such minibodies are administered, and thus in clinical applications minibodies are advantageous over whole antibodies such as IgG. It is thus expected that minibodies can be used as anti-cancer agents with superior characteristics as compared to previous agonist antibodies. Furthermore, minibodies are not glycosylated, and thus their expression systems are not limited when they are expressed as recombinant proteins. For example, minibodies can be produced using various expression systems such as mammalian cell lines, yeasts, insect cells, and *E. coli.* In addition, the present invention demonstrated that minibodies with multivalent antigen binding sites, in particular trivalent or more antigen binding sites, are particularly effective as agonist antibodies against receptors such as TRAIL receptors that form trimers and transmit signals.

## Claims

1. An antibody that recognizes a tumor necrosis factor-related apoptosis-inducing ligand receptor (TRAIL receptor).

2. The antibody of claim 1, which is a minibody.

3. The antibody of claim 1 or 2, which comprises three or more antigen binding sites.

4. The antibody of claim 3, which comprises three antigen binding sites.

5. The antibody of claim 4, wherein three scFv units form a trimer.

6. The antibody of claim 5, wherein two of the variable regions in the scFv units are linked together *via* a linker with zero to two amino acids.

7. The antibody of claim 6, wherein the linker comprises zero amino acids.

8. The antibody of claim 6, wherein the linker comprises one amino acid.

9. The antibody of claim 3, which comprises four antigen binding sites.

10. The antibody of claim 9, wherein a polypeptide comprising four variable regions forms a dimer.

11. The antibody of any one of claims 1 to 10, wherein the TRAIL receptor is TRAIL-R1 or TRAIL-R2.

12. The antibody of any one of claims 1 to 11, which induces apoptosis in a cell.

13. The antibody of claim 12, wherein the cell is a tumor cell.

14. An antibody comprising the amino acid sequence of SEQ ID NO: 2.

15. An antibody comprising the amino acid sequence of SEQ ID NO: 4.

16. An antibody comprising the amino acid sequence of SEQ ID NO: 6.

17. An antibody comprising the amino acid sequence of SEQ ID NO: 8.

18. An antibody that comprises three or more antigen binding sites and induces apoptosis in a cell.

19. The antibody of claim 18, which comprises three antigen binding sites.

20. The antibody of claim 18, which comprises four antigen binding sites.

21. The antibody of any one of claims 18 to 20, wherein the cell is a tumor cell.

22. A polynucleotide encoding the antibody of any one of claims 1 to 21.

23. A polynucleotide that hybridizes to the polynucleotide of claim 22 under stringent conditions and encodes an antibody with an activity equivalent to that of the antibody of any one of claims 1 to 21.

24. A vector carrying the polynucleotide of claim 22 or 23.

25. A host cell carrying the polynucleotide of claim 22 or 23, or the vector of claim 24.

26. A pharmaceutical composition comprising the antibody of any one of claims 1 to 21.


SEQUENCE LISTING

<110>  CHUGAI SEIYAKU KABUSHIKI KAISHA

<120>  Modified antibodies recognizing trimer receptor or higher

<130>  C1-A0324P

<150>  JP 2003-415735
<151>  2003-12-12

<160>  42

<170>  PatentIn version 3.1

<210>  1
<211>  797
<212>  DNA
<213>  Artificial

<220>
<223>  An artificially synthesized nucleotide sequence

<400>  1
tagaattcca ccatggagtt tgggctgagc tggctttttc ttgtggctat tttaaaaggt      60

gtccagtgtg aggtacagct gttggagtct gggggaggct tggtacagcc tgggaggtcc     120

ctgagactct cctgtgcagc ctctggattc acctttagca gctatgccat gagctgggtc     180

cgccaggctc cagggaaggg gctggagtgg gtctcagcta ttagtggtag tggtggtagc     240

agatactacg cagactccgt gaagggccgg ttcaccatct ccagagacaa ttccaagaac     300

acgctgtatc tgcaaatgaa cagcctgaga gccgaggaca cggccgtata ttactgtgcg     360

aaagagagca gtggctggtt cggggccttt gactactggg gccagggaac cctggtcacc     420

gtctcctcag gtggagaaat tgtgctgact cagtctccag actttcagtc tgtgactcca     480

aaggagaaag tcaccatcac ctgccgggcc agtcagagca ttggtagtag cttacactgg     540

taccagcaga aaccagatca gtctccaaag ctcctcatca agtatgcttc ccagtccttc     600

tcaggggtcc cctcgaggtt cagtggcagt ggatctggga cagatttcac cctcaccatc     660

aatagcctgg aagctgaaga tgctgcagcg tattactgtc atcagagtag tagtttaccg     720

atcaccttcg gccaagggac acgactggag attaaagact acaaggatga cgacgataag     780

tgataagcgg ccgcaat                                                    797

<210>  2
<211>  256
<212>  PRT
<213>  Artificial

<220>
<223>  An artificially synthesized peptide sequence

<400>  2
Met Glu Phe Gly Leu Ser Trp Leu Phe Leu Val Ala Ile Leu Lys Gly
1               5                   10                  15

Val Gln Cys Glu Val Gln Leu Leu Glu Ser Gly Gly Gly Leu Val Gln
            20                  25                  30

Pro Gly Arg Ser Leu Arg Leu Ser Cys Ala Ala Ser Gly Phe Thr Phe

35                  40                  45

Ser Ser Tyr Ala Met Ser Trp Val Arg Gln Ala Pro Gly Lys Gly Leu
    50                  55                  60

Glu Trp Val Ser Ala Ile Ser Gly Ser Gly Gly Ser Arg Tyr Tyr Ala
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Lys Glu Ser Ser Gly Trp Phe Gly Ala Phe Asp Tyr
        115                 120                 125

Trp Gly Gln Gly Thr Leu Val Thr Val Ser Ser Gly Gly Glu Ile Val
    130                 135                 140

Leu Thr Gln Ser Pro Asp Phe Gln Ser Val Thr Pro Lys Glu Lys Val
145                 150                 155                 160

Thr Ile Thr Cys Arg Ala Ser Gln Ser Ile Gly Ser Ser Leu His Trp
                165                 170                 175

Tyr Gln Gln Lys Pro Asp Gln Ser Pro Lys Leu Leu Ile Lys Tyr Ala
            180                 185                 190

Ser Gln Ser Phe Ser Gly Val Pro Ser Arg Phe Ser Gly Ser Gly Ser
        195                 200                 205

Gly Thr Asp Phe Thr Leu Thr Ile Asn Ser Leu Glu Ala Glu Asp Ala
    210                 215                 220

Ala Ala Tyr Tyr Cys His Gln Ser Ser Ser Leu Pro Ile Thr Phe Gly
225                 230                 235                 240

Gln Gly Thr Arg Leu Glu Ile Lys Asp Tyr Lys Asp Asp Asp Asp Lys
                245                 250                 255


<210>  3
<211>  794
<212>  DNA
<213>  Artificial

<220>
<223>  An artificially synthesized nucleotide sequence

<400>  3
tagaattcca ccatggagtt tgggctgagc tggctttttc ttgtggctat tttaaaaggt      60

gtccagtgtg aggtacagct gttggagtct gggggaggct tggtacagcc tggggggtcc     120

ctgagactct cctgtgcagc ctctggattc acctttagca gctatgccat gagctgggtc     180

cgccaggctc cagggaaggg gctggagtgg gtctcagcta ttagtggtag tggtggtagc     240

agatactacg cagactccgt gaagggccgg ttcaccatct ccagagacaa ttccaagaac     300

acgctgtatc tgcaaatgaa cagcctgaga gccgaggaca cggccgtata ttactgtgcg     360

aaagagagca gtggctggtt cggggccttt gactactggg gccagggaac cctggtcacc     420

gtctcctcag gtgaaattgt gctgactcag tctccagact ttcagtctgt gactccaaag     480

gagaaagtca ccatcacctg ccgggccagt cagagcattg gtagtagctt acactggtac     540

cagcagaaac cagatcagtc tccaaagctc ctcatcaagt atgcttccca gtccttctca     600

ggggtcccct cgaggttcag tggcagtgga tctgggacag atttcaccct caccatcaat     660

agcctggaag ctgaagatgc tgcagcgtat tactgtcatc agagtagtag tttaccgatc     720

accttcggcc aagggacacg actggagatt aaagactaca aggatgacga cgataagtga     780

taagcggccg caat                                                       794

<210>  4
<211>  255
<212>  PRT
<213>  Artificial

<220>
<223>  An artificially synthesized peptide sequence

<400>  4
Met Glu Phe Gly Leu Ser Trp Leu Phe Leu Val Ala Ile Leu Lys Gly
1               5                   10                  15

Val Gln Cys Glu Val Gln Leu Leu Glu Ser Gly Gly Gly Leu Val Gln
            20                  25                  30

Pro Gly Arg Ser Leu Arg Leu Ser Cys Ala Ala Ser Gly Phe Thr Phe
        35                  40                  45

Ser Ser Tyr Ala Met Ser Trp Val Arg Gln Ala Pro Gly Lys Gly Leu
    50                  55                  60

Glu Trp Val Ser Ala Ile Ser Gly Ser Gly Gly Ser Arg Tyr Tyr Ala
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Lys Glu Ser Ser Gly Trp Phe Gly Ala Phe Asp Tyr
        115                 120                 125

Trp Gly Gln Gly Thr Leu Val Thr Val Ser Ser Gly Glu Ile Val Leu
    130                 135                 140

Thr Gln Ser Pro Asp Phe Gln Ser Val Thr Pro Lys Glu Lys Val Thr
145                 150                 155                 160

Ile Thr Cys Arg Ala Ser Gln Ser Ile Gly Ser Ser Leu His Trp Tyr
                165                 170                 175

Gln Gln Lys Pro Asp Gln Ser Pro Lys Leu Leu Ile Lys Tyr Ala Ser
            180                 185                 190

Gln Ser Phe Ser Gly Val Pro Ser Arg Phe Ser Gly Ser Gly Ser Gly
        195                 200                 205

Thr Asp Phe Thr Leu Thr Ile Asn Ser Leu Glu Ala Glu Asp Ala Ala
    210                 215                 220

Ala Tyr Tyr Cys His Gln Ser Ser Ser Leu Pro Ile Thr Phe Gly Gln
225                 230                 235                 240

Gly Thr Arg Leu Glu Ile Lys Asp Tyr Lys Asp Asp Asp Asp Lys
                245                 250                 255

<210> 5
<211> 791
<212> DNA
<213> Artificial

<220>
<223> An artificially synthesized nucleotide sequence

<400> 5
tagaattcca ccatggagtt tgggctgagc tggctttttc ttgtggctat tttaaaaggt 60

gtccagtgtg aggtacagct gttggagtct gggggaggct tggtacagcc tgggaggtcc 120

ctgagactct cctgtgcagc ctctggattc acctttagca gctatgccat gagctgggtc 180

cgccaggctc cagggaaggg gctggagtgg gtctcagcta ttagtggtag tggtggtagc 240

agatactacg cagactccgt gaagggccgg ttcaccatct ccagagacaa ttccaagaac 300

acgctgtatc tgcaaatgaa cagcctgaga gccgaggaca cggccgtata ttactgtgcg 360

aaagagagca gtggctggtt cggggccttt gactactggg gccagggaac cctggtcacc 420

gtctcctcag aaattgtgct gactcagtct ccagactttc agtctgtgac tccaaaggag 480

aaagtcacca tcacctgccg ggccagtcag agcattggta gtagcttaca ctggtaccag 540

cagaaaccag atcagtctcc aaagctcctc atcaagtatg cttcccagtc cttctcaggg 600

gtcccctcga ggttcagtgg cagtggatct gggacagatt tcaccctcac catcaatagc 660

ctggaagctg aagatgctgc agcgtattac tgtcatcaga gtagtagttt accgatcacc 720

ttcggccaag ggacacgact ggagattaaa gactacaagg atgacgacga taagtgataa 780

gcggccgcaa t 791

<210> 6
<211> 254
<212> PRT
<213> Artificial

<220>
<223> An artificially synthesized peptide sequence

<400> 6
Met Glu Phe Gly Leu Ser Trp Leu Phe Leu Val Ala Ile Leu Lys Gly
1               5                   10                  15

Val Gln Cys Glu Val Gln Leu Leu Glu Ser Gly Gly Gly Leu Val Gln
            20                  25                  30

Pro Gly Arg Ser Leu Arg Leu Ser Cys Ala Ala Ser Gly Phe Thr Phe
        35                  40                  45

Ser Ser Tyr Ala Met Ser Trp Val Arg Gln Ala Pro Gly Lys Gly Leu
    50                  55                  60

Glu Trp Val Ser Ala Ile Ser Gly Ser Gly Gly Ser Arg Tyr Tyr Ala
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Lys Glu Ser Ser Gly Trp Phe Gly Ala Phe Asp Tyr

        115                 120                 125
Trp Gly Gln Gly Thr Leu Val Thr Val Ser Ser Glu Ile Val Leu Thr
    130                 135                 140
Gln Ser Pro Asp Phe Gln Ser Val Thr Pro Lys Glu Lys Val Thr Ile
145                 150                 155                 160
Thr Cys Arg Ala Ser Gln Ser Ile Gly Ser Ser Leu His Trp Tyr Gln
                165                 170                 175
Gln Lys Pro Asp Gln Ser Pro Lys Leu Leu Ile Lys Tyr Ala Ser Gln
            180                 185                 190
Ser Phe Ser Gly Val Pro Ser Arg Phe Ser Gly Ser Gly Ser Gly Thr
        195                 200                 205
Asp Phe Thr Leu Thr Ile Asn Ser Leu Glu Ala Glu Asp Ala Ala Ala
    210                 215                 220
Tyr Tyr Cys His Gln Ser Ser Ser Leu Pro Ile Thr Phe Gly Gln Gly
225                 230                 235                 240
Thr Arg Leu Glu Ile Lys Asp Tyr Lys Asp Asp Asp Asp Lys
                245                 250

<210> 7
<211> 1538
<212> DNA
<213> Artificial

<220>
<223> An artificially synthesized nucleotide sequence

<400> 7
tagaattcca ccatggagtt tgggctgagc tggctttttc ttgtggctat tttaaaaggt 60
gtccagtgtg aggtacagct gttggagtct gggggaggct tggtacagcc tgggaggtcc 120
ctgagactct cctgtgcagc ctctggattc acctttagca gctatgccat gagctgggtc 180
cgccaggctc cagggaaggg gctggagtgg gtctcagcta ttagtggtag tggtggtagc 240
agatactacg cagactccgt gaagggccgg ttcaccatct ccagagacaa ttccaagaac 300
acgctgtatc tgcaaatgaa cagcctgaga gccgaggaca cggccgtata ttactgtgcg 360
aaagagagca gtggctggtt cggggccttt gactactggg gccagggaac cctggtcacc 420
gtctcctcag gtggaggcgg atcggaaatt gtgctgactc agtctccaga ctttcagtct 480
gtgactccaa aggagaaagt caccatcacc tgccgggcca gtcagagcat tggtagtagc 540
ttacactggt accagcagaa accagatcag tctccaaagc tcctcatcaa gtatgcttcc 600
cagtccttct caggggtccc ctcgaggttc agtggcagtg gatctgggac agatttcacc 660
ctcaccatca atagcctgga agctgaagat gctgcagcgt attactgtca tcagagtagt 720
agtttaccga tcaccttcgg ccaagggaca cgactggaga ttaaaagagc tgatgctgca 780
gctgcaggag gtcccgggtc cgaggtacag ctgttggagt ctgggggagg cttggtacag 840
cctgggaggt ccctgagact ctcctgtgca gcctctggat tcacctttag cagctatgcc 900
atgagctggg tccgccaggc tccagggaag gggctggagt gggtctcagc tattagtggt 960
agtggtggta gcagatacta cgcagactcc gtgaagggcc ggttcaccat ctccagagac 1020

aattccaaga acacgctgta tctgcaaatg aacagcctga gagccgagga cacggccgta    1080

tattactgtg cgaaagagag cagtggctgg ttcggggcct ttgactactg gggccaggga    1140

accctggtca ccgtctcctc aggtggaggc ggatcggaaa ttgtgctgac tcagtctcca    1200

gactttcagt ctgtgactcc aaaggagaaa gtcaccatca cctgccgggc cagtcagagc    1260

attggtagta gcttacactg gtaccagcag aaaccagatc agtctccaaa gctcctcatc    1320

aagtatgctt cccagtcctt ctcaggggtc ccctcgaggt tcagtggcag tggatctggg    1380

acagatttca ccctcaccat caatagcctg gaagctgaag atgctgcagc gtattactgt    1440

catcagagta gtagtttacc gatcaccttc ggccaaggga cacgactgga gattaaagac    1500

tacaaggatg acgacgataa gtgataagcg gccgcaat                            1538

<210>  8
<211>  503
<212>  PRT
<213>  Artificial

<220>
<223>  An artificially synthesized peptide sequence

<400>  8
Met Glu Phe Gly Leu Ser Trp Leu Phe Leu Val Ala Ile Leu Lys Gly
1               5                   10                  15

Val Gln Cys Glu Val Gln Leu Leu Glu Ser Gly Gly Gly Leu Val Gln
            20                  25                  30

Pro Gly Arg Ser Leu Arg Leu Ser Cys Ala Ala Ser Gly Phe Thr Phe
        35                  40                  45

Ser Ser Tyr Ala Met Ser Trp Val Arg Gln Ala Pro Gly Lys Gly Leu
    50                  55                  60

Glu Trp Val Ser Ala Ile Ser Gly Ser Gly Gly Ser Arg Tyr Tyr Ala
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Lys Glu Ser Ser Gly Trp Phe Gly Ala Phe Asp Tyr
        115                 120                 125

Trp Gly Gln Gly Thr Leu Val Thr Val Ser Ser Gly Gly Gly Gly Ser
    130                 135                 140

Glu Ile Val Leu Thr Gln Ser Pro Asp Phe Gln Ser Val Thr Pro Lys
145                 150                 155                 160

Glu Lys Val Thr Ile Thr Cys Arg Ala Ser Gln Ser Ile Gly Ser Ser
                165                 170                 175

Leu His Trp Tyr Gln Gln Lys Pro Asp Gln Ser Pro Lys Leu Leu Ile
            180                 185                 190

Lys Tyr Ala Ser Gln Ser Phe Ser Gly Val Pro Ser Arg Phe Ser Gly
        195                 200                 205

Ser Gly Ser Gly Thr Asp Phe Thr Leu Thr Ile Asn Ser Leu Glu Ala

210                 215                 220

Glu Asp Ala Ala Ala Tyr Tyr Cys His Gln Ser Ser Ser Leu Pro Ile
225                 230                 235                 240

Thr Phe Gly Gln Gly Thr Arg Leu Glu Ile Lys Arg Ala Asp Ala Ala
                245                 250                 255

Ala Ala Gly Gly Pro Gly Ser Glu Val Gln Leu Leu Glu Ser Gly Gly
            260                 265                 270

Gly Leu Val Gln Pro Gly Arg Ser Leu Arg Leu Ser Cys Ala Ala Ser
        275                 280                 285

Gly Phe Thr Phe Ser Ser Tyr Ala Met Ser Trp Val Arg Gln Ala Pro
    290                 295                 300

Gly Lys Gly Leu Glu Trp Val Ser Ala Ile Ser Gly Ser Gly Gly Ser
305                 310                 315                 320

Arg Tyr Tyr Ala Asp Ser Val Lys Gly Arg Phe Thr Ile Ser Arg Asp
                325                 330                 335

Asn Ser Lys Asn Thr Leu Tyr Leu Gln Met Asn Ser Leu Arg Ala Glu
            340                 345                 350

Asp Thr Ala Val Tyr Tyr Cys Ala Lys Glu Ser Ser Gly Trp Phe Gly
        355                 360                 365

Ala Phe Asp Tyr Trp Gly Gln Gly Thr Leu Val Thr Val Ser Ser Gly
    370                 375                 380

Gly Gly Gly Ser Glu Ile Val Leu Thr Gln Ser Pro Asp Phe Gln Ser
385                 390                 395                 400

Val Thr Pro Lys Glu Lys Val Thr Ile Thr Cys Arg Ala Ser Gln Ser
                405                 410                 415

Ile Gly Ser Ser Leu His Trp Tyr Gln Gln Lys Pro Asp Gln Ser Pro
            420                 425                 430

Lys Leu Leu Ile Lys Tyr Ala Ser Gln Ser Phe Ser Gly Val Pro Ser
        435                 440                 445

Arg Phe Ser Gly Ser Gly Ser Gly Thr Asp Phe Thr Leu Thr Ile Asn
    450                 455                 460

Ser Leu Glu Ala Glu Asp Ala Ala Ala Tyr Tyr Cys His Gln Ser Ser
465                 470                 475                 480

Ser Leu Pro Ile Thr Phe Gly Gln Gly Thr Arg Leu Glu Ile Lys Asp
                485                 490                 495

Tyr Lys Asp Asp Asp Asp Lys
            500


<210>  9
<211>  15
<212>  DNA
<213>  Artificial

<220>
<223>  An artificial sequence encoding linker sequence

<400>  9
ggtggaggcg gatcg                                                    15

<210> 10
<211> 5
<212> PRT
<213> Artificial

<220>
<223> An artificially synthesized linker sequence

<400> 10
Gly Gly Gly Gly Ser
1               5


<210> 11
<211> 24
<212> DNA
<213> Artificial

<220>
<223> An artificial sequence encoding flag tag sequence

<400> 11
gactacaagg atgacgacga taag 24


<210> 12
<211> 8
<212> PRT
<213> Artificial

<220>
<223> An artificially synthesized flag tag sequence

<400> 12
Asp Tyr Lys Asp Asp Asp Asp Lys
1               5


<210> 13
<211> 806
<212> DNA
<213> Artificial

<220>
<223> An artificially synthesized diabody sequence

<400> 13
tagaattcca ccatggagtt tgggctgagc tggctttttc ttgtggctat tttaaaaggt 60

gtccagtgtg aggtacagct gttggagtct gggggaggct tggtacagcc tgggaggtcc 120

ctgagactct cctgtgcagc ctctggattc acctttagca gctatgccat gagctgggtc 180

cgccaggctc cagggaaggg gctggagtgg gtctcagcta ttagtggtag tggtggtagc 240

agatactacg cagactccgt gaagggccgg ttcaccatct ccagagacaa ttccaagaac 300

acgctgtatc tgcaaatgaa cagcctgaga gccgaggaca cggccgtata ttactgtgcg 360

aaagagagca gtggctggtt cggggccttt gactactggg gccagggaac cctggtcacc 420

gtctcctcag gtggaggcgg atcggaaatt gtgctgactc agtctccaga ctttcagtct 480

gtgactccaa aggagaaagt caccatcacc tgccgggcca gtcagagcat tggtagtagc 540

ttacactggt accagcagaa accagatcag tctccaaagc tcctcatcaa gtatgcttcc 600

cagtccttct caggggtccc ctcgaggttc agtggcagtg gatctgggac agatttcacc 660

ctcaccatca atagcctgga agctgaagat gctgcagcgt attactgtca tcagagtagt 720

agtttaccga tcaccttcgg ccaagggaca cgactggaga ttaaagacta caaggatgac 780

gacgataagt gataagcggc cgcaat 806

<210> 14
<211> 94
<212> DNA
<213> Artificial

<220>
<223> An artificially synthesized oligonucleotide sequence

<400> 14
tagaattcca ccatggagtt tgggctgagc tggctttttc ttgtggctat tttaaaaggt 60

gtccagtgtg aggtacagct gttggagtct gggg 94

<210> 15
<211> 96
<212> DNA
<213> Artificial

<220>
<223> An artificially synthesized oligonucleotide sequence

<400> 15
tgctaaaggt gaatccagag gctgcacagg agagtctcag ggacctccca ggctgtacca 60

agcctccccc agactccaac agctgtacct cacact 96

<210> 16
<211> 97
<212> DNA
<213> Artificial

<220>
<223> An artificially synthesized oligonucleotide sequence

<400> 16
cctgtgcagc ctctggattc acctttagca gctatgccat gagctgggtc cgccaggctc 60

cagggaaggg gctggagtgg gtctcagcta ttagtgg 97

<210> 17
<211> 99
<212> DNA
<213> Artificial

<220>
<223> An artificially synthesized oligonucleotide sequence

<400> 17
ttggaattgt ctctggagat ggtgaaccgg cccttcacgg agtctgcgta gtatctgcta 60

ccaccactac cactaatagc tgagacccac tccagcccc 99

<210> 18
<211> 103
<212> DNA
<213> Artificial

<220>

<223>  An artificially synthesized oligonucleotide sequence

<400>  18
ccggttcacc atctccagag acaattccaa gaacacgctg tatctgcaaa tgaacagcct      60

gagagccgag gacacggccg tatattactg tgcgaaagag agc                       103


<210>  19
<211>  87
<212>  DNA
<213>  Artificial

<220>
<223>  An artificially synthesized oligonucleotide sequence

<400>  19
ggagacggtg accagggttc cctggcccca gtagtcaaag gccccgaacc agccactgct      60

ctctttcgca cagtaatata cggccgt                                          87


<210>  20
<211>  98
<212>  DNA
<213>  Artificial

<220>
<223>  An artificially synthesized oligonucleotide sequence

<400>  20
tggggccagg gaaccctggt caccgtctcc tcaggtggag gcggatcgga aattgtgctg      60

actcagtctc cagactttca gtctgtgact ccaaagga                              98


<210>  21
<211>  79
<212>  DNA
<213>  Artificial

<220>
<223>  An artificially synthesized oligonucleotide sequence

<400>  21
taagctacta ccaatgctct gactggcccg gcaggtgatg gtgactttct cctttggagt      60

cacagactga aagtctgga                                                   79


<210>  22
<211>  103
<212>  DNA
<213>  Artificial

<220>
<223>  An artificially synthesized oligonucleotide sequence

<400>  22
cgggccagtc agagcattgg tagtagctta cactggtacc agcagaaacc agatcagtct      60

ccaaagctcc tcatcaagta tgcttcccag tccttctcag ggg                       103


<210>  23
<211>  97
<212>  DNA
<213>  Artificial

<220>
<223>  An artificially synthesized oligonucleotide sequence

<400>  23
gcttccaggc tattgatggt gagggtgaaa tctgtcccag atccactgcc actgaacctc      60

gaggggaccc ctgagaagga ctgggaagca tacttga                               97


<210>  24
<211>  90
<212>  DNA
<213>  Artificial

<220>
<223>  An artificially synthesized oligonucleotide sequence

<400>  24
tttcaccctc accatcaata gcctggaagc tgaagatgct gcagcgtatt actgtcatca      60

gagtagtagt ttaccgatca ccttcggcca                                       90


<210>  25
<211>  93
<212>  DNA
<213>  Artificial

<220>
<223>  An artificially synthesized oligonucleotide sequence

<400>  25
attgcggccg cttatcactt atcgtcgtca tccttgtagt ctttaatctc cagtcgtgtc      60

ccttggccga aggtgatcgg taaactacta ctc                                   93


<210>  26
<211>  26
<212>  DNA
<213>  Artificial

<220>
<223>  An artificially synthesized primer sequence

<400>  26
tagaattcca ccatggagtt tgggct                                           26


<210>  27
<211>  26
<212>  DNA
<213>  Artificial

<220>
<223>  An artificially synthesized primer sequence

<400>  27
ggagacggtg accagggttc cctggc                                           26


<210>  28
<211>  26
<212>  DNA
<213>  Artificial

<220>
<223>  An artificially synthesized primer sequence

<400>  28
tggggccagg gaaccctggt caccgt                                          26


<210>  29
<211>  26
<212>  DNA
<213>  Artificial

<220>
<223>  An artificially synthesized primer sequence

<400>  29
attgcggccg cttatcactt atcgtc                                          26


<210>  30
<211>  35
<212>  DNA
<213>  Artificial

<220>
<223>  An artificially synthesized primer sequence

<400>  30
tcctcaggtg gagaaattgt gctgactcag tctcc                                35


<210>  31
<211>  36
<212>  DNA
<213>  Artificial

<220>
<223>  An artificially synthesized primer sequence

<400>  31
aatttctcca cctgaggaga cggtgaccag ggttcc                               36


<210>  32
<211>  32
<212>  DNA
<213>  Artificial

<220>
<223>  An artificially synthesized primer sequence

<400>  32
tcctcaggtg aaattgtgct gactcagtct cc                                   32


<210>  33
<211>  36
<212>  DNA
<213>  Artificial

<220>
<223>  An artificially synthesized primer sequence

<400>  33
cacaatttca cctgaggaga cggtgaccag ggttcc                               36


<210>  34
<211>  32
<212>  DNA
<213>  Artificial

<220>
<223>  An artificially synthesized primer sequence

<400>  34
gtctcctcag aaattgtgct gactcagtct cc                                    32


<210>  35
<211>  36
<212>  DNA
<213>  Artificial

<220>
<223>  An artificially synthesized primer sequence

<400>  35
cacaatttct gaggagacgg tgaccagggt tccctg                                36


<210>  36
<211>  12
<212>  PRT
<213>  Artificial

<220>
<223>  An artificially synthesized linker sequence

<400>  36
Arg Ala Asp Ala Ala Ala Ala Gly Gly Pro Gly Ser
1               5                   10


<210>  37
<211>  60
<212>  DNA
<213>  Artificial

<220>
<223>  An artificially synthesized primer sequence

<400>  37
ggacccggga cctcctgcag ctgcagcatc agctctttta atctccagtc gtgtcccttg      60


<210>  38
<211>  35
<212>  DNA
<213>  Artificial

<220>
<223>  An artificially synthesized primer sequence

<400>  38
ggtcccgggt ccgaggtaca gctgttggag tctgg                                 35


<210>  39
<211>  37
<212>  DNA
<213>  Artificial

<220>
<223>  An artificially synthesized primer sequence

<400>  39
gataagcttc caccatggag tttgggctga gctggct                               37


<210>  40

<211>  43
<212>  DNA
<213>  Artificial

<220>
<223>  An artificially synthesized primer sequence

<400>  40
gtcggatcca ctcacctgag gagacggtga ccagggttcc ctg                43

<210>  41
<211>  94
<212>  DNA
<213>  Artificial

<220>
<223>  An artificially synthesized primer sequence

<400>  41
gataagcttc caccatgtcg ccatcacaac tcattgggtt tctgctgctc tgggttccag      60

cctccagggg tgaaattgtg ctgactcagt ctcc                                  94

<210>  42
<211>  40
<212>  DNA
<213>  Artificial

<220>
<223>  An artificially synthesized primer sequence

<400>  42
gtcggatcca ctcacgttta atctccagtc gtgtcccttg                            40